# EUROPEAN PATENT APPLICATION

(11) **EP 4 303 234 A1**
(43) Date of publication of application: **10.01.2024**
(21) Application number: 22762624.9
(22) Date of filing: 04.03.2022
(51) Int. Cl.: C07K 16/28, A61K 39/395, A61K 38/17, A61K 39/00

(54) **ANTIBODY AGAINST NKP46 AND APPLICATION OF ANTIBODY**

(30) Priority: 05.03.2021 CN 202110243601
(71) Applicant: Shanghai Qilu Pharmaceutical Research and Development Centre Ltd., Shanghai 201203 (CN)
(72) Inventor: YANG, Liuqing, Shanghai 201203 (CN); GU, Jinming, Shanghai 201203 (CN)
(74) Representative: Elzaburu S.L.P.
(86) International application number: PCT/CN2022/079236
(87) International publication number: WO 2022/184162

(57) **Abstract**

Provided are an anti-NKp46 antibody or antigen-binding fragment thereof, a derivative comprising said antibody or antigen-binding fragment thereof, a pharmaceutical composition, and related application in treating cancers.

## Description

### TECHNICAL FIELD

The present disclosure belongs to the field of immunology. In particular, the present disclosure relates to an antibody against the NKp46 receptor or antigen-binding fragments thereof, a derivative comprising the antibody or antigen-binding fragment thereof, a pharmaceutical composition, and related application thereof in the treatment of cancer.

### BACKGROUND

NK cells are effector lymphocytes in the innate immune system, and are the body's first line of defense against viral infections and tumorigenesis. They play a similar role with cytotoxic T cells in the acquired immune system. However, the differences between them are that: it usually needs to detect MHC on the surface of viral cells, cytotoxic T cells can induce the release of cytokines, thereby leading to lysis or apoptosis of target cells; while NK cells can recognize these cells and respond quickly in conditions without antibodies or MHC. For those cells that lost own markers thereof such as MHC class I molecules, NK cells can kill them without activation, and these cells are usually harmful, but cannot be detected and destroyed by other immune cells.

NK cells regulate cell activity through a balance of activating receptors and inhibitory receptors on the surface. The activating receptors on the surface of NK cells are divided into human leukocyte antigen class I (HLA-I) molecular-associated receptors and non-associated receptors, the former includes killer cell immunogloblin like receptor, two Ig domains and short cytoplasmic tail 2 (KIR2DS), killer cell immunoglobulin like receptor, three Ig domains and short cytoplasmic tail 3 (KIR3DS) and natural killer cell group 2C (CD94/NKG2C); the latter mainly includes natural killer cell group 2D (2DNKG2D) and natural cytotoxicity receptors (NCR; including NKp46, NKp30, NKp44) and DNAX accessory molecule-1 (DNAM-1); the corresponding inhibitory receptors are KIR2DL, KIR3DL, CD94/NKG2A, etc.

The NKp46 receptor discovered by Sivori et al. in 1997 is the first natural cytotoxic receptor and also one of the most important natural cytotoxic receptor, it is expressed on the surface of all NK cells (including mature, immature, quiescent, and activated NK cells), and plays a pivotal role in natural killing. It is specifically expressed on the surface of NK cells with a molecular weight of 46 kD, hence named as NKp46. The NKp46 gene is located on chromosome 19, and NKp46 is a type I transmembrane glycoprotein. The extracellular region of NKp46 contains 2 Ig-like domains, namely D1 and D2, where D2 (near the cell membrane region) is the ligand-binding region of tumor and virus-infected cells. The transmembrane region has a positively charged arginine and a cytoplasmic tail lacking an immune receptor tyrosine-based activation sequence, which can form a salt bridge with aspartate residues in CD3ζ and FcyR transmembrane region, the latter allows tyrosine phosphorylation by binding to receptor, and thus mediates signal transduction of NKp46. So far, the ligands identified for NKp46 only includes influenza virus hemagglutinin and parainfluenza virus hemagglutinin, while cellular ligands are unknown.

When NK cells kill target cells, toxic particles shall reach the surface of plasma membrane and fuse with the cell membrane, thereby causing the release of granule contents and ultimately leading to the death of the target cells. As degranulation occurs, CD107a molecules are transported to the cell membrane surface, and the upregulated expression of CD107a molecules is consistent with perforin secretion. Therefore, CD107a molecule-positive NK cells may represent NK cells with killer activity. Role of NKp46 in antitumor: Activated NK cells have a strong tumor cell killing effect, while NKp46 receptors are required to deliver activation stimuli. It has been known that decreased expression of NKp46 involves immune escape from cervical cancer and its prodromal lesions, and loss of NKp46/NCR1 expression increases lymphoma growth. Yasser et al. confirmed that NKp46 is necessary for the killing of all MM cell lines. In addition, NKp46 also plays an important role in the removal of microglia in the central nervous system. Lunemann et al. confirmed that, NK cells infiltrated in the brain allow activated NK cells to form synaptic connections to microglia through NKp46, NKG2D-mediated recognition, and play a role in killing when the latter displays perforin polarization toward the cell interface.

### SUMMARY OF THE INVENTION

The present disclosure provides an anti-NKp46 antibody or antigen-binding fragment thereof, the anti-NKp46 antibody or antigen-binding fragment thereof specifically binds to human and cynomolgus monkey NKp46.

In some embodiments, the present disclosure provides an anti-NKp46 antibody or antigen-binding fragment thereof. The anti-NKp46 antibody or antigen-binding fragment thereof is capable of specifically binding to NKp46, comprises 3 CDRs selected from sequences shown in SEQ ID NOs: 44-133.

In some embodiments, the present disclosure provides an anti-NKp46 antibody or antigen-binding fragment thereof. The anti-NKp46 antibody or antigen-binding fragment thereof is capable of specifically binding to NKp46, and comprises: HCDR1 selected from a group consisting of SEQ ID NOs: 44, 47, 50, 53, 56, 59, 62, 65, 68, 71, 74, 77, 80, 83, 86, 89, 92, 95, 98, 101, 104, 107, 110, 113, 116, 119, 122, 125, 128, 131; and HCDR2 selected from a group consisting of SEQ ID NOs: 45, 48, 51, 54, 57, 60, 63, 66, 69, 72, 75, 78, 81, 84, 87, 90, 93, 96, 99, 102, 105, 108, 111, 114, 117, 120, 123, 126, 129, 132; and HCDR3 selected from a group consisting of SEQ ID NOs: 46, 49, 52, 55, 58, 61, 64, 67, 70, 73, 76, 79, 82, 85, 88, 91, 94, 97, 100, 103, 106, 109, 112, 115, 118, 121, 124, 127, 130, 133.

In some embodiments, the present disclosure provides an anti-NKp46 antibody or antigen-binding fragment thereof, The anti-NKp46 antibody or antigen-binding fragment thereof is capable of specifically binding to NKp46, and comprises a heavy chain variable region, where the heavy chain variable region comprises HCDR1, HCDR2 and HCDR3 selected from the following groups: SEQ ID NO: 44, SEQ ID NO: 45 and SEQ ID NO: 46; or SEQ ID NO: 47, SEQ ID NO: 48 and SEQ ID NO: 49; or SEQ ID NO: 50, SEQ ID NO: 51 and SEQ ID NO: 52; or SEQ ID NO: 53, SEQ ID NO: 54 and SEQ ID NO: 55; or SEQ ID NO: 56, SEQ ID NO: 57 and SEQ ID NO: 58; or SEQ ID NO: 59, SEQ ID NO: 60 and SEQ ID NO: 61; or SEQ ID NO: 62, SEQ ID NO: 63 and SEQ ID NO: 64; or SEQ ID NO: 65, SEQ ID NO: 66 and SEQ ID NO: 67; or SEQ ID NO: 68, SEQ ID NO: 69 and SEQ ID NO: 70; or SEQ ID NO: 71, SEQ ID NO: 72 and SEQ ID NO: 73; or SEQ ID NO: 74, SEQ ID NO: 75 and SEQ ID NO: 76; or SEQ ID NO: 77, SEQ ID NO: 78 and SEQ ID NO: 79; or SEQ ID NO: 80, SEQ ID NO: 81 and SEQ ID NO: 82; or SEQ ID NO: 83, SEQ ID NO: 84 and SEQ ID NO: 85; or SEQ ID NO: 86, SEQ ID NO: 87 and SEQ ID NO: 88; or SEQ ID NO: 89, SEQ ID NO: 90 and SEQ ID NO:91; or SEQ ID NO: 92, SEQ ID NO: 93 and SEQ ID NO: 94; or SEQ ID NO: 95, SEQ ID NO: 96 and SEQ ID NO: 97; or SEQ ID NO: 98, SEQ ID NO: 99 and SEQ ID NO: 100; or SEQ ID NO:101, SEQ ID NO:102 and SEQ ID NO:103; or SEQ ID NO:104, SEQ ID NO: 105 and SEQ ID NO: 106; or SEQ ID NO: 107, SEQ ID NO: 108 and SEQ ID NO: 109; or SEQ ID NO: 110, SEQ ID NO: 111 and SEQ ID NO: 112; or SEQ ID NO: 113, SEQ ID NO: 114 and SEQ ID NO: 115; or SEQ ID NO: 116, SEQ ID NO: 117 and SEQ ID NO: 118; or SEQ ID NO: 119, SEQ ID NO: 120 and SEQ ID NO: 121; or SEQ ID NO: 122, SEQ ID NO: 123 and SEQ ID NO: 124; or SEQ ID NO: 125, SEQ ID NO: 126 and SEQ ID NO: 127; or SEQ ID NO: 128, SEQ ID NO: 129 and SEQ ID NO: 130; or SEQ ID NO: 131, SEQ ID NO: 132 and SEQ ID NO: 133.

In some embodiments, the present disclosure provides an anti-NKp46 antibody or antigen-binding fragment thereof, wherein the anti-NKp46 antibody or antigen-binding fragment thereof is capable of specifically binding to NKp46, and comprises HCDR1, HCDR2 and HCDR3 selected from the heavy chain variable regions shown in SEQ ID NOs: 14-43, 136-152.

In some embodiments, the present disclosure provides an anti-NKp46 antibody or antigen-binding fragment thereof, wherein the anti-NKp46 antibody or antigen-binding fragment thereof is capable of specifically binding to NKp46, and comprises a heavy chain variable region which has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NOs: 14-43, 136-152.

In some embodiments, the anti-NKp46 antibody or antigen-binding fragment thereof provided herein is a monoclonal antibody, wherein the monoclonal antibody is a recombinant antibody, preferably, an alpaca-derived antibody, a chimeric antibody or a humanized antibody.

In some embodiments, the present disclosure provides the anti-NKp46 antibody or antigen-binding fragment thereof according to the present disclosure, wherein the monoclonal antibody is a nanobody; preferably, the antibody is a humanized camelid VHH.

In some embodiments, the anti-NKp46 antibody or antigen-binding fragment thereof provided in the present disclosure further comprises a heavy chain constant region and/or a light chain constant region, preferably, the heavy chain constant region comprises Fc or variant Fc, the Fc is derived from mice or humans.

In some embodiments, the anti-NKp46 antibody or antigen-binding fragment thereof provided in the present disclosure is in the formats of IgG1, IgG2, IgG3, or IgG4.

In some embodiments, the present disclosure provides a conjugate according to the present disclosure, which is formed by coupling any of the preceding anti-NKp46 antibodies or antigen-binding fragments thereof to a capture label or a detection label, the detection label comprises radionuclides, luminescent substances, colored substances, or enzymes.

In some embodiments, the present disclosure provides a bispecific antibody, wherein an antigen-binding domain comprises any of the preceding anti-NKp46 antibodies or antigen-binding fragments thereof.

In some embodiments, the present disclosure provides a multi-specific antibody, wherein an antigen-binding domain comprises any of the preceding anti-NKp46 antibodies or antigen-binding fragments thereof.

In some embodiments, the present disclosure provides an antibody-drug conjugate comprising any of the preceding anti-NKp46 antibodies or antigen-binding fragments thereof, the antibody-drug conjugate is formed by antibody-linker-toxin interconnections.

In some embodiments, the present disclosure provides a chimeric antigen receptor, in which extracellular recognition unit comprises any of the preceding anti-NKp46 antibodies or antigen-binding fragments thereof.

In some embodiments, the present disclosure provides a nucleic acid encoding any of the preceding anti-NKp46 antibodies or antigen-binding fragments thereof.

In some embodiments, the present disclosure provides a recombinant vector according to the present disclosure.

In some embodiments, the present disclosure provides a host cell comprising the recombinant vector described in the present disclosure or an integrated genome comprising the nucleic acid described in the present disclosure.

In some embodiments, the present disclosure provides a method of preparing the anti-NKp46 antibody or antigen-binding fragment thereof, comprising: culturing the host cells described in the present disclosure under suitable conditions, and purifying expression products obtained from the cells.

In some embodiments, the present disclosure provides use of the anti-NKp46 antibody or antigen-binding fragment thereof described in the present disclosure in the preparation of drugs for specifically targeting NKp46-expressing cells, preferably, the cells are NK cells.

In some embodiments, the present disclosure provides a use of the anti-NKp46 antibody or antigen-binding fragment thereof described in the present disclosure in the preparation of drugs for cancers, infectious diseases, or inflammatory or autoimmune diseases, preferably, the cancers include: leukemia, aggressive lymphoma, non-Hodgkin lymphoma, glioma, cervical cancer, head and neck cancer, rectal cancer, kidney cancer, liver cancer, lung cancer, pancreatic cancer, stomach cancer, etc.

In some embodiments, the present disclosure provides a use of the anti-NKp46 antibody or antigen-binding fragment thereof described in the present disclosure in the preparation of diagnostic reagents for NKp46-expressing NK cells.

In some embodiments, the present disclosure provides a solution preparation, comprising an anti-NKp46 antibody or antigen-binding fragment thereof, and buffer solution.

In some embodiments, the present disclosure provides a method for identifying the presence of NKp46-expressing cells in an individual, the method comprises obtaining a biological sample from the individual containing cells, contacting the cells with the anti-NKp46 antibody or its antigen-binding fragment thereof described in the present disclosure, and assessing the binding of the antibody to the cells or not.

In some embodiments, the present disclosure provides a pharmaceutical composition comprising an effective amount of any of the preceding anti-NKp46 antibodies or antigen-binding fragments thereof, or comprising an effective amount of the conjugate, or comprising an effective amount of the bispecific antibody, or comprising an effective amount of the multi-specific antibody, or comprising an effective amount of the antibody-drug conjugate, or comprising an effective amount of the chimeric antigen receptor, or comprising an effective amount of the nucleic acid, or comprising an effective amount of the recombinant vector, or comprising an effective amount of the host cell.

In some embodiments, the pharmaceutical composition provided in the present disclosure further comprises a pharmaceutically acceptable carrier.

In some embodiments, the pharmaceutical composition provided in the present disclosure further comprises one or more additional therapeutic agents; preferably, the one or more additional therapeutic agents include: chemotherapeutic agents, cytotoxic agents, radiotherapeutic agents, cancer vaccines, anti-neoplastic agents, targeted anti-cancer agents, antiangiogenic agents, biological response modifiers, cytokines, hormones, anti-metastatic agents, and immunotherapeutic agents.

In some embodiments, the present disclosure provides a medicine box or a kit, comprising a container and the pharmaceutical composition described in the present disclosure and held in the container.

### BRIEF DESCRIPTION OF THE DRAWINGS

The drawings attached herein further illustrate the novel features disclosed in this specification. The features and advantages disclosed in this specification will be better understood with reference to the drawings, but it should be understood that these drawings are merely illustrative for specific embodiments of the principles disclosed herein, and are not intended to limit the scope of the attached claims.
FIG. 1 shows the expression of human NKp46 on CHOK1 cells validated by anti-NKp46 antibody as positive control, as detected by flow cytometry; wherein the solid line represents the expression of human NKp46 on CHOK1 maternal cells without being transfected any plasmid, and the dotted line in the figure represents the overexpression of human NKp46 protein after CHOK1 maternal cells were transfected with human NKp46 plasmid.
FIG. 2 shows the expression of cynomolgus monkey NKp46 on CHOK1 cells validated by anti-NKp46-antibody as positive control, as detected by flow cytometry; wherein the solid line represents the expression of cynomolgus monkey NKp46 on CHOK1 maternal cells without being transfected any plasmid, and the dotted line in the figure shows the overexpression of cynomolgus monkey NKp46 protein after CHOK1 maternal cells were transfected with cynomolgus monkey NKp46 plasmid.
FIG. 3-6 show the binding of anti-NKP46 chimeric antibody of the present disclosure to NKp46-expressing cells (human NKp46-CHOK1).
FIG. 7-10 show the binding of anti-NKp46 chimeric antibody of the present disclosure to NKp46-expressing cells (cynomolgus monkey NKp46-CHOK1).
FIG. 11-12 show the in vitro NK cell activation by anti-NKp46 chimeric antibody of the present disclosure.
FIG. 13-16 show the binding of anti-NKp46 humanized antibody of the present disclosure to NKp46-expressing cells (human NKp46-CHOK1).
FIG. 17-20 show the binding of anti-NKp46 humanized antibody of the present disclosure to NKp46-expressing cells (cynomolgus monkey NKp46-CHOK1).

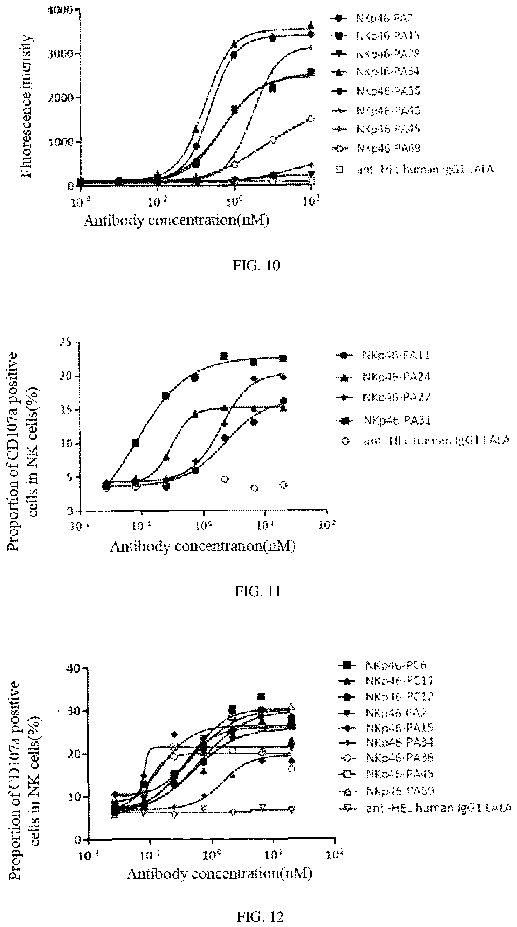

FIG. 11-12 show the in vitro NK cell activation by anti-NKp46 humanized antibody of the present disclosure.

### DETAILED DESCRIPTION OF EMBODIMENTS

### TERMS

All publications, patents, and patent applications mentioned in this specification are incorporated herein by reference, the extent of reference is as if each publication, patent, or patent application was specifically and individually incorporated by reference.

Before the present disclosure is described in detail below, it is to be understood that, the present disclosure is not limited to the particular methodology, protocols, and reagents described herein, as they may vary. It is also to be understood that the terms used herein are only to describe embodiments, not intended to limit the scope of the present disclosure. Unless otherwise specified, all technical and scientific terms used herein have the same meanings as those generally understood by a person of ordinary skill in the art to which the present disclosure belongs.

Certain embodiments disclosed herein encompass numerical ranges, and certain aspects of the present disclosure may be described in terms of ranges. Unless otherwise indicated, it is to be understood that, these numerical ranges or the description in the form of ranges are merely for brevity and convenience, and should not be construed as strict limits on the scope of the present disclosure. Accordingly, the description in the form of ranges should be considered to specifically disclose all possible subranges and all possible specific numerical points within the range, as such subranges and numerical points are expressly written herein. For example, a description in a range of from 1 to 6 should be considered to specifically disclose subranges from 1 to 3, 1 to 4, 1 to 5, 2 to 4, 2 to 6, 3 to 6, etc., as well as specific numerical points within these ranges, e.g., 1, 2, 3, 4, 5, 6. The above principles are equally applicable regardless of the width of the numerical ranges. Where a range description is employed, the range includes the endpoints of the range.

The term "about", when it relates to a measurable value such as an amount and temporal duration, refers to a change that includes ±20%, or in some cases ±10%, or in some cases ±5%, or in some cases ±1%, or in some cases ±0.1% of the specified value.

Three-letter codes and one-letter codes for amino acid as used herein are as described in J. Biol. Chem, 243, p3558(1968).

Conventional immunoglobulins are tetramers consisting of two heavy and two light chains, which has a combined molecular weight of about 150 kDa. In the camelidae family member, a significant percentage of serum antibodies are homodimer IgG with a molecular weight of about 80 kD (Hamers-Casterman, et al. 1993, Nature, 363, 446-448). These heavy-chain immunoglobulins (Ig) contain three domains, variable regions thereof are called variable domain of heavy chain of heavy-chain antibody (VHH). Recombinant VHH (approximately 12 to 14 kD) constitutes a complete antigen-binding domain and shows a broad antigen-binding profile, thereby expanding its highly variable regions and showing unique properties, for example, residues in three to four hydrophobic frames (which interacts with VL of conventional antibodies) are replaced by more hydrophilic amino acids. To stabilize the expanded CDR, in addition to conventional disulfide bonds, VHH may have additional disulfide bonds between CDR1 and CDR3 in camelus dromedaries, between CDR2 and CDR3 in llamas (Harmsen and De Haard. 2007, Appl Microbiol Biotechnol., 77, 13-22; Muyldermans. 2001, J Biotechnol., 74, 277-302). Enlarged CDR3 rings can take convex conformations, while conventional complementary bits are confined in concave or planar structures (Muyldermans, 2001, J Biotechnol., 74, 277-302). These features allow VHH to recognize unique epitopes that have poor immunogenicity for conventional antibodies (Lafaye, et al., 2009, Mol Immuno., 46, 695-704; Wernery, 2001, J Vet Med B Infect Dis Vet Public Health., 48, 561-568). Although VHH is defined as a monovalent antibody that excludes any effect of affinity by default, the measured biological activity of IC₅₀ in vitro can be similar to that of conventional bivalent antibody molecules (Thys et al., 2010 Antiviral Res., 87: 257-264).

The term "monoclonal antibody" refers to antibodies obtained from a population of substantially homologous antibodies, i.e., each antibody contained in the population are identical (except for possible natural mutations that may be present in a small amount). Monoclonal antibodies are highly specific for a single antigen site. In addition, it is different from conventional polyclonal antibody preparations (which typically include different antibodies against different determinants (epitopes) on the antigen) that each monoclonal antibody is only specific to a single determinant or epitope on the antigen.

In some embodiments, the present disclosure may involve chimeric camelid-human antibodies, in particular, where VH and/or VL domains are entirely sequences of camelids (e.g., Llama or Alpaca), and the remainder of the antibodies are completely human sequences. In some preferred embodiments of the present disclosure, it further comprises camelid antibodies and camelid/human chimeric antibodies by "humanization" or "germline", wherein the VH and/or VL domains contain one or more amino acid substitutions in the framework region with respect to the camelid VH and/or VL domains obtained by active immunity. The "humanization" process of replacing mismatched amino acid residues in the original camelid VH or VL domains with corresponding residues in human germline VH or VL domain, thus such "humanization" process increases the percentage of sequence identity with the human germline VH or VL domain.

The present disclosure includes natural, recombinant VHH or VH.

The "recombinant" involves the use of genetically engineered methods (cloning, amplification) to produce the VHH or VH.

The VHH according to the present disclosure may be in the form of a monomer or in the form of a homologous polymer, such as homodimer or homotrimer.

The antibodies of the present disclosure include alpaca-derived antibodies, chimeric antibodies, humanized antibodies, preferably humanized antibodies.

The "chimeric antibody", which is an antibody fused by the variable region of an alpaca-derived antibody with the constant region (or Fc region) of human antibody, can reduce the immune response induced by an alpaca-derived antibody. To construct a chimeric antibodies, it is necessary to establish a hybridoma or antibody library secreting alpaca-derived specific monoclonal antibodies first, and then link the alpaca antibody variable region gene with the human constant region gene (or Fc region gene) into a chimeric gene, insert it into a expression vector, and finally express the chimeric antibody molecule in the eukaryotic system or prokaryotic system.

The "humanized antibody" refers to an antibody generated in different types of human germline antibody framework sequence by transplanting the CDR sequence of alpaca antibody into the framework of the variable region of human antibody. It is possible to overcome the heterologous response induced by the chimeric antibody due to carrying a large amount of alpaca protein components. Such framework sequences can be obtained from public DNA databases or published references including germline antibody gene sequences. Germline DNA sequences such as human heavy-chain and light-chain variable region genes are available in the human germline sequence database "VBase"

(https://www2.mrc-lmb.cam.ac.uk/vbase/), as well as in the paper of Kabat, E.A., et al., 1991, Sequences of Proteins of Immunological Interest, 5th edition. To avoid a decrease in activity of antibody caused by a decrease in immunogenicity, the human antibody variable region framework sequence can be subjected to minimal reverse mutation or back mutation to maintain the activity.

The "VHH" involves variable antigen-binding domains from camelid heavy chain antibodies (camel, dromedary, liama, alpaca, etc.) (see Nguyen et al., 2000, EMBO J., 19, 921-930; Muyldermans, 2001, J Biotechnol., 74, 277-302, and the review Vanlandschoot et al., 2011, Antiviral Res.92, 389-407) .

Generally, nanobodies can be defined as amino acid sequences with the following (general) structure: FR1-CDR1-FR2-CDR2-FR3-CDR3-FR4. Wherein FR1-FR4 refer to frame regions 1-4 respectively, and CDR1-CDR3 refer to complementarity determining regions 1-3 respectively.

As known in the art, antibodies are classified into five categories: IgA, IgD, IgE, IgG, and IgM, corresponding heavy chain constant domains thereofare termed a, δ, , ε, γ and µ, respectively. IgG and IgA may be further classified into different subclasses, for example, IgG may be classified into IgGl, IgG2, IgG3, and IgG4, and IgA may be classified into IgAl and IgA2. The light chains of antibodies from any vertebrate species can be assigned to one of the two distinct types, called κ and λ, based on the amino acid sequences of constant domains.

In the antibodies of IgG, IgA, and IgD, the constant region comprises three domains called CH1, CH2, CH3 (IgM and IgE have the fourth domain called CH4). In the subclasses of IgG, IgA, and IgD, the CH1 and CH2 domains are separated by a flexible hinge region, is the hinge region is a proline-rich and cysteine-rich segment with a variable length. Each type of antibodies further comprise interchain and intrachain disulfide bonds formed by paired cysteine residues.

The term "Fc" is used herein to define a C-terminal region of an immunoglobulin heavy chain, the region comprises at least a portion of the constant region. This term includes a native sequence Fc region or a variant Fc region. Unless otherwise indicated, numbering of amino acid residues in the Fc region or constant region is made according to the EU numbering system, which is also referred to as the EU index, as described in Kabat et al., Sequences of Proteins of Immunological Interest, 5th Ed. Public Health Service, National Institutes of Health, Bethesda, Md., 1991.

It should be noted that the division of CDR and FR of variable region of monoclonal antibody disclosed in the present disclosure is determined according to the Kabat definition. Other naming and numbering systems, such as Chothia, IMGT or AHo etc. are also known to those skilled in the art. Accordingly, based on the monoclonal antibody sequences of the present disclosure, humanized antibodies containing one or more CDRs derived from any of naming systems are explicitly maintained within the scope of the present disclosure.

The term "sequence identity" or "sequence similarity", or "sequence homology" refers to the percentage of amino acid residues in a candidate sequence to the same amino acid residues in a reference polypeptide sequence, after the sequences are aligned (and gaps are introduced when necessary) to achieve the maximum percent sequence identity, and no conservative substitutions are considered as part of the sequence identity. Sequence alignments can be performed using various approaches in the art to determine percent amino acid sequence identity, for example, using publicly available computer software such as BLAST, BLAST-2, ALIGN, or MEGALIGN (DNASTAR) software. Those skilled in the art can determine appropriate parameters for alignment measurement, including any algorithm required to achieve the maximum alignment over the full length of the sequences being compared.

The term "antibody fragment" encompasses at least a portion of an intact antibody. As used herein, the fragment of an antibody molecule includes an "antigen-binding fragment" of the antibody, and the term "antigen-binding fragment" refers to a polypeptide fragment of an immunoglobulin or antibody that specifically binds to or reacts with a selected antigen or antigenic epitope thereof, or a fusion protein product further derived from the fragment, e.g., a single-chain antibody, an extracellular binding region in a chimeric antigen receptor, etc. Exemplary antibody fragments or antigen-binding fragments thereof include, but are not limited to light chain variable fragments (VL), heavy chain variable fragments (VH), Fab fragments, F(ab')2 fragments, Fd fragments, Fv fragments, single domain antibodies, linear antibodies, single-chain antibodies (scFv), and bispecific antibodies or multi-specific antibodies formed from antibody fragments, etc.

The term "multi-specific antibody" refers to a new antibody construct that binds to more than two different sites and/or targets, which is formed by functionally linking to an antibody (e.g., chemical conjugation, gene fusion, non-covalent binding, or other methods) to one or more other binding molecules. Among them, the most used one is "bispecific antibody", which specifically refers to an antibody construct that is specific for two different antigens. Typically, bispecific or multi-specific antibodies include at least 2 antigen-binding domains.

The term "antigen" refers to a substance recognized and specifically bound by an antibody or antigen binding fragment, and broadly, an antigen can include any immunogenic fragment or determinant of a selected target, including a single epitope, multiple epitopes, a single domain, multiple domains, or an entire extracellular domain (ECD) or a protein. Peptides, proteins, glycoproteins, polysaccharides and lipids, portions and combinations thereof may constitute antigens. Non-limiting exemplary antigens include tumor antigens or pathogen antigens, etc. "Antigens" may also refer to molecules that trigger an immune response. Any form of antigen or cells or preparations containing the antigen can be used to generate antibodies specific to antigen determinants. The antigen can be isolated full-length proteins, cell surface proteins (e.g., immunized with cells expressing at least a portion of the antigen on the surface thereof), or soluble proteins (e.g., immunized only with the ECD portion of the protein), or protein constructs (e.g., Fc antigens). The antigen may be produced in genetically modified cells. Any of the foregoing antigens may be used alone or in combination with one or more immunogenicity-enhancing adjuvants known in the art. The DNA encoding the antigen may be genomic or non-genomic (e.g., cDNA) and may encode at least a portion of the ECD sufficient to trigger an immunogenic response. Any vector may be used to transform cells in which the antigen is expressed, the vector includes, but not limited to adenoviral vectors, lentiviral vectors, plasmids, and non-viral vectors such as cationic lipids.

The term "epitope" refers to a site on an antigen that specifically binds to an immunoglobulin or antibody. Epitopes may be formed from contiguous amino acids, or from noncontiguous amino acids juxtaposed by tertiary folding of a protein. Epitopes formed from contiguous amino acids are typically retained upon exposure to denaturing solvents, while epitopes formed by tertiary folding are typically lost upon treatment with denaturing solvents. Epitopes typically comprise at least 3-15 amino acids in a unique spatial conformation. Methods for determining the epitope to which a given antibody binds are well known in the art, including immunoblotting and immunoprecipitation detection assays, etc. Methods for determining the spatial conformation of an epitope include techniques in the art and described herein, such as X-ray crystallography, two-dimensional nuclear magnetic resonance, etc.

The terms "polypeptide", "peptide", and "protein" are used interchangeably herein to refer to polymers of amino acids with any length. The polymer may be linear, cyclic, or branched, and may comprise modified amino acids, particularly conservatively modified amino acids, and it may be interrupted by non-amino acids. The term also includes modified amino acid polymers, for example, amino acid polymers are modified by sulfation, glycosylation, lipidation, acetylation, phosphorylation, iodination, methylation, oxidation, proteolytic processing, prenylation, racemization, selenylation, transfer RNA (tRNA)-mediated amino addition such as arginylation, ubiquitination, or any other operation such as conjugation to a labeling component. As used herein, the term "amino acid" refers to natural and/or non-natural or synthetic amino acids, including glycine and D or L optical isomers, as well as amino acid analogs and peptidomimetics. A polypeptide or amino acid sequence "derived from" a given protein refers to the source of the polypeptide. The term also includes polypeptides expressed by the specified nucleic acid sequences.

The term "amino acid modification" (or "modified amino acid") includes amino acid substitutions, insertions, and/or deletions in a polypeptide sequence. As used herein, "amino acid substitution" or "substitution" refers to the replacement of an amino acid at a particular position in a parent polypeptide sequence with another amino acid. For example, the S32A substitution means that serine at position 32 is replaced with alanine.

Sequence identity or homology of a humanized antibody variable region to a human receptor variable region can be determined as discussed herein, and when measured in this way, the two will preferably share at least 60% or 65% sequence identity, more preferably at least 70%, 75%, 80%, 85% or 90% sequence identity, even more preferably at least 93%, 95%, 98% or 99% sequence identity. Preferably, residue positions that are not identical differ by conservative amino acid substitutions. The "conservative substitution" is an amino acid substitution in which one amino acid residue is replaced with another amino acid residue having a side chain (R group) with similar chemical properties (e.g., electric charge or hydrophobicity). In general, conservative amino acid substitutions do not substantially alter the functional properties of proteins. Families of amino acid residues having similar side chains have been defined in the art. These families include amino acids containing basic side chains (e.g., lysine, arginine, histidine), acidic side chains (e.g., aspartic acid, glutamic acid), uncharged polar side chains (e.g., glycine, asparagine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, one or more amino acid residues in the CDR regions or framework regions of the antibodies in the present disclosure may be replaced with amino acid residues having other similar side chains. In the case where two or more amino acid sequences differ from one another by conservative substitutions, the percent sequence identity or degree of similarity may be adjusted upward to correct the conservative nature of the substitution.

The term "affinity" or "binding affinity" refers to the strength of a sum of all non-covalent interactions between a single binding site of a molecule (e.g., an antibody) and its binding partner (e.g., an antigen). The term "K_{D}" refers to the dissociation constant of a particular antibody-antigen interaction. Various techniques known in the art can be used to determine the binding affinity, such as surface plasmon resonance, biolayer interferometry, dual polarization interferometry, static light scattering, dynamic light scattering, isothermal titration calorimetry, ELISA, analytical ultracentrifugation and flow cytometry. The term "competitive binding" or "competitive antibody" generally refers to the degree of competitive inhibition which can be obtained in the competition assay, where the binding of the antibodies (which has the same epitope as the antibody of the present disclosure) to the epitope causes that the binding between the antibody of the present disclosure and the epitope is inhibited or blocked.

The term "pharmaceutical composition" refers to a formulation present in a form that allows the biological activity of active ingredients contained therein to be effective and does not contain additional ingredients having unacceptable toxicity to subjects to which the formulation is administered.

The term "pharmaceutically carrier" or "pharmaceutically acceptable carrier" refers to a diluent, adjuvant (e.g., Freund's adjuvant (complete and incomplete)), excipient, or vehicle with which a therapeutic agent is administered.

The term "effective amount" refers to a dose of a pharmaceutical formulation of an antibody or fragment of the present disclosure that produces desired effects in treated patients after administration to patients in a single dose or multiple doses. An effective amount can be readily determined by the attending physician as one skilled in the art, by considering the following factors: for example, the differences of human species; body weight, age, and health conditions; specific diseases; disease severity; response of an individual patient; specific antibody administered; modes of administration; bioavailability characteristics of the administered formulation; a selected dosing regimen; and use of any concomitant therapy.

The terms "host cell", "host cell line" and "host cell culture" are used interchangeably and refer to a cell into which an exogenous nucleic acid is introduced, including progeny of such a cell. Host cells include "transformants" and "transformed cells", which include primarily transformed cells and progeny derived therefrom, regardless of the number of passages. The progeny may not be the same as the parent cell in nucleic acid contents, but may contain mutations. Mutant progeny having the same function or biological activity as screened or selected in the initially transformed cell are included herein.

The term "transfection" used herein refers to the introduction of an exogenous nucleic acid into a eukaryotic cell. Transfection can be accomplished by various means known in the art, including calcium phosphate-DNA co-precipitation, DEAF-dextran mediated transfection, polybrene-mediated transfection, electroporation, microinjection, liposome fusion, lipid transfection, protoplast fusion, retroviral infection, and biolistics.

The term "stable transfection" or "ST" refers to the introduction and integration of an exogenous nucleic acid, DNA, or RNA into the genome of a transfected cell. The term "stable transfectant" refers to a cell that stably integrates foreign DNA into genomic DNA.

The terms "nucleic acid molecule encoding", "coding DNA sequence" and "coding DNA" refer to the order of deoxyribonucleotides along a strand of deoxyribonucleic acid. The order of these deoxyribonucleotides determines the order of amino acids along the polypeptide (protein) chain. Thus, the nucleic acid sequence encodes an amino acid sequence.

Methods of producing and purifying antibodies or antigen-binding fragments thereof are known in prior art and can be found, and found in prior art, such as Cold Spring Harbor Protocols, Guidelines for Antibody Experimental Technology, Chapters 5-8 and 15. Antibodies or antigen-binding fragments thereof described in the present disclosure are produced by genetically engineering methods to add one or more human FR regions into a non-human CDR region. The human germline FR sequence can be available on the ImMunoGeneTics(IMGT) website (http://imgt.cines.fr) or from the Immunoglobulin FactsBook (2001) ISBN: 012441351).

The engineered antibodies or antigen-binding fragments thereof of the present disclosure can be prepared and purified by conventional methods. For example, cDNA sequences encoding heavy and light chains can be cloned and recombined into expression vectors. The recombinant immunoglobulin expression vector can stably transfect CHO cells. As a more commonly recommended prior art, mammalian expression systems may result in glycosylation of antibodies, particularly at the highly conserved N-terminus of the Fc region. Stable clones are obtained by expressing antibodies that specifically bind to human antigens. Positive clones are expandingly cultured in a serum-free medium in a bioreactor to produce antibodies. The antibody-secreting medium may be purified and collected using conventional techniques. The antibody may be concentrated by filtration using conventional methods. Soluble mixtures and polymers may also be removed by conventional methods, such as molecular sieves, ion exchange, etc.

As used herein, the term "individual" or "subject" refers to any animal, such as mammal or marsupial. Individuals of the present disclosure include but are not limited to, humans, non-human primates (e.g., cynomolgus monkey or rhesus monkey or other types of macaques), mice, pigs, horses, donkeys, cattle, sheep, rats, and any kind of poultry.

As used herein, the term "tumor" refers to a disease characterized by pathological proliferation of cells or tissues, and subsequent migration or invasion of other tissues or organs. The growth of tumor is usually uncontrolled and progressive, but does not induce or inhibit normal cell proliferation. Tumors can affect various cells, tissues or organs, including, but not limited to, bladder, bone, brain, breast, cartilage, glial cells, esophagus, fallopian tube, gallbladder, heart, intestine, kidney, liver, lung, lymph nodes, nerve tissue, ovary, pancreas, prostate, skeletal muscle, skin, spinal cord, spleen, stomach, testis, thymus, thyroid, trachea, urethra, ureter, urethra, uterus, vaginal organ, or tissue or corresponding cell. Tumors include cancers, such as sarcomas, carcinomas, or plasmacytomas (malignant tumors of plasma cells). The tumor according to the present disclosure may include, but is not limited to, leukemia (e.g. acute leukemia, acute lymphocytic leukemia, acute myeloid leukemia, acute granulocytic leukemia, acute promyelocytic leukemia, acute myelomonocytic leukemia, acute monocytic leukemia, chronic leukemia, chronic myelogenous leukemia, chronic lymphocytic leukemia, polycythemia vera), lymphoma (Hodgkin's disease, non-Hodgkin's disease), primary macroglobulinemia, heavy chain disease, solid tumors such as sarcomas and cancers (e.g. fibrosarcoma, myxosarcoma, liposarcoma, chondrosarcoma, osteosarcoma, chordoma, endothelial sarcoma, lymphangiosarcoma, angiosarcoma, lymphangioendothelioma, mesothelioma, Ewing's tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat adenocarcinoma, sebaceous adenocarcinoma, papillary carcinoma, papillary adenocarcinoma, bronchial carcinoma, myeloid cancer, renal cell carcinoma, liver cancer, bile duct carcinoma, choriocarcinoma, seminoma, embryo cancer, nephroblastoma, cervical cancer, uterine cancer, testicular cancer, lung cancer, small cell lung cancer, bladder cancer, epithelial cancer, glioma, astrocytoma, medulloblastoma, craniopharyngioma, ependymoma, pinealoma, hemangioblastoma, acoustic neuromas, oligodendroglioma, schwannoma, meningioma, melanoma, neuroblastoma, retinoblastoma), esophageal cancer, gallbladder cancer, kidney cancer, multiple myeloma. Preferably, the "tumor" includes, but limited to: pancreatic cancer, liver cancer, lung cancer, gastric cancer, esophageal cancer, head and neck squamous cell carcinoma, prostate cancer, colon cancer, breast cancer, lymphoma, gallbladder cancer, renal cancer, leukemia, multiple myeloma, ovarian cancer, cervical cancer, and glioma.

The term "disease" or "condition" or "disorder" or the like used herein refers to any alteration or disorder that impairs or interferes with the normal function of a cell, tissue, or organ. For example, the "disease" includes, but is not limited to: tumors, pathogen infections, autoimmune diseases, T-cell dysfunctions, or deficiencies in immune tolerance (e.g., transplant rejection).

The term "treatment" used herein refers to clinical intervention in an attempt to alter a disease caused by an individual or treated cells, either prophylactically or clinically pathologically. Therapeutic effects include but are not limited to, prevention of the occurrence or recurrence of a disease, alleviation of symptoms, reduction of any disease's direct or indirect pathological consequences, prevention of metastasis, slowing of the rate of disease progression, amelioration or remission of a condition, remission or amelioration of a prognosis, etc.

The term "medicine box" or "kit" includes an effective amount of one or more unit dosage forms of a pharmaceutical composition of the present disclosure. In some embodiments, the medicine box or kit may include sterile containers; such containers may be in the form of boxes, ampoules, bottles, vials, tubes, bags, blister packs, or other suitable containers known in the art. Such containers may be made of plastic, glass, laminated paper, metal foil, or other materials suitable for storing medicaments. In addition, the medicine box also includes instructions for administering the pharmaceutical composition of the present disclosure to individuals. The instructions generally include methods of using the pharmaceutical compositions of the present disclosure to treat diseases.

### EXAMPLES

The present disclosure will be further described in detail below in combination with specific examples. It should be understood that, these examples are only used to describe the present disclosure, but are not intended to limit the scope of the present disclosure. The experimental methods in the following examples which are not specified with specific conditions are generally carried out according to conventional conditions (J. Sambrook et al., Molecular Cloning: A Laboratory Manual (Third Edition), Science Press, 2002, or according to the conditions recommended by the manufacturer.

### Example 1: Antigen information of human NKp46 and cynomolgus monkey NKp46

**Table 1. Sequence information**

| Name | Sequence number | Note |
|---|---|---|
| Full-length amino acid sequence of human NKp46 | SEQ ID NO: 1 | The region at positions 1-21 is a signal peptide, the region at positions 22-258 is NKp46 extracellular region, the region at positions 259-279 is a transmembrane region, and the region at positions 280-304 is an intracellular region. |
| Full-length amino acid sequence of cynomolgus monkey NKp46 | SEQ ID NO: 2 | The region at positions 1-21 are a signal peptide, the region at positions 22-257 is NKp46 extracellular region, the region at positions 258-278 is a transmembrane region, and the region at positions 279-306 is an intracellular region. |
| Human NKp46 antigen for immunization, screening, and testing (Kactus, NKP-HM146) | SEQ ID NO: 3 | - |
| Cynomolgus monkey NKp46 for immunization, screening, and testing (Kactus, NKP-CM146) | SEQ ID NO: 4 | - |

### Example 2: Preparation of human NKp46 and cynomolgus monkey NKp46 cell lines

The nucleotide sequence encoding human NKp46 amino acids shown in SEQ ID NO: 1 was cloned into a pCMV3 (SinoBiological, Cat. number CV011) vector for construction of a human NKp46 cell line. The obtained vector was transfected into CHOK1 cells (ATCC, Cat. number CCL-61) to obtain a CHOK1 cell line expressing human NKp46 (abbreviated as human NKp46-CHOK1 cell line).

The nucleotide sequence encoding cynomolgus monkey NKp46 amino acids shown in SEQ ID NO: 2 was cloned into the pCMV3 vector to obtain the vector for the construction of a cynomolgus monkey NKp46 cell line . The obtained vector was transfected into CHOK1 cells to obtain a CHOK1 cell line expressing cynomolgus monkey NKp46 (abbreviated as cynomolgus NKp46-CHOK1 cell line).

Using a positive control antibody (Santa Cruz Biotechnology, Cat. number sc-59343), the expression of human NKp46 in the human NKp46-CHOK1 cell line obtained above was detected using the FACS assay, and a negative control anti-HEL human IgG1 LALA (Biointron, Cat. number B109802) was set in the experiment.

Using a positive control antibody (Santa Cruz Biotechnology, Cat. number sc-59343), the expression of cynomolgus monkey NKp46 in the above obtained cynomolgus monkey NKp46-CHOK1 cell line was detected, and a negative control anti-HEL human IgG1 LALA (Biointron, Cat. number B109802) was set in the experiment.

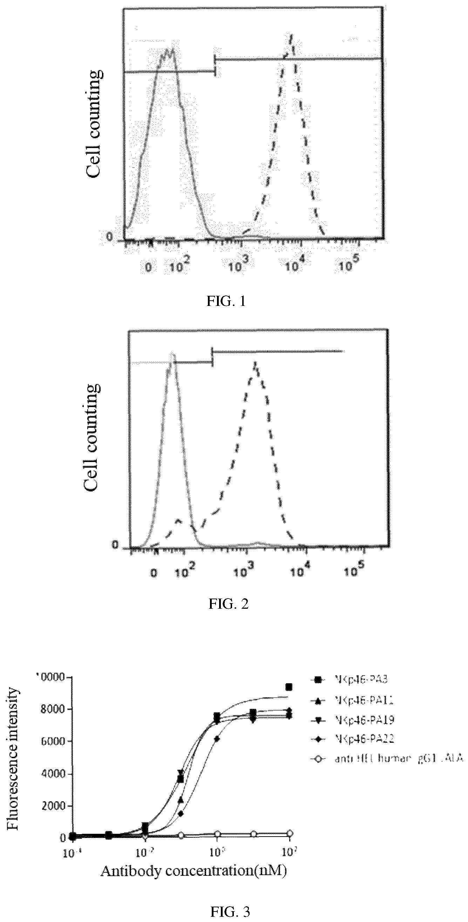

The results show:
The expression of human NKp46 in the human NKp46-CHOK1 cell line is shown in Figure 1, and the results show that human NKp46 has good overexpression in CHOK1 cells, which can be used for subsequent experiments to verify the binding of NKp46 monoclonal antibody to human NKp46 at the cellular level.

The expression of cynomolgus monkey NKp46 in cynomolgus monkey NKp46-CHOK1 cell line is shown in Figure 2, and the results show that cynomolgus monkey NKp46 has good overexpression in CHOK1 cells, which can be used for subsequent experiments to verify the binding of cynomolgus monkey NKp46 monoclonal antibody to cynomolgus monkey NKp46 at the cellular level.

### Example 3: Construction of immune alpaca/camel VHH heavy-chain antibody library

Alpacas/camels were immunized at intervals with human NKp46 antigen shown in SEQ ID NO: 3 and cynomolgus monkey NKp46 antigen as shown in SEQ ID NO: 4, respectively. The dates of immunization were set on days 0, 14, 8, and 42, for a total of 4 immunizations. Serum was isolated by blood samples on days 28, 42, and 56, and the immune response in serum was measured by flow cytometry. When the serum titer was greater than 1:10000, the immunization would be ended. 100 mL of blood samples from are retaken from immunized alpacas/camels respectively as follows:
1. Using lymphocyte isolate solution (Solarbio Company), separate camel/alpaca PBMC according to the protocol thereof.
2. Using the Cell Total RNA Extraction Kit (OMEGA Company), extract total RNA of camel/alpaca separately according to the protocol thereof.
3. Using the PrimeScript^{™} II Reverse Transcription Kit (Takara Company), synthesis cDNA of camel/alpaca according to the protocol thereof.
4. Perform Nested PCR amplification using the specific primers shown in SEQ ID NOs: 5-13 and using the cDNA obtained above as a template.
   First-round PCR
      SEQ ID NO: 5 (Forward primer): GTCCTGGCTGCTCTTCTACAAGG.
      SEQ ID NO: 6 (Reverse primer): GGTACGTGCTGTTGAACTGTTCC.
   Second-round PCR
      SEQ ID NO: 7 (Forward primer):
         ACTCGCGGCCCAGCCGGCCATGGCCGAGGTGCAGCTGGTGGAGTCTGGGGGAG.
      SEQ ID NO: 8 (Forward primer):
         ACTCGCGGCCCAGCCGGCCATGGCCCAGGTRCAGCTGGTGGAGTCTGGGGGAG.
      SEQ ID NO: 9 (Forward primer):
         5 ACTCGCGGCCCAGCCGGCCATGGCCGATGTGCAGCTGGTGGAGTCTGGGGGAG.
      SEQ ID NO: 10 (Reverse primer):
         GGTGTTGGCCTCCCGGGCCACTAGTGCTKGAGACRGTGACCTGGGT.
      SEQ ID NO: 11 (Reverse primer)::
         GGTGTTGGCCTCCCGGGCCACTAGTGCTKGAGACRGTGACCAGGGT.
      SEQ ID NO: 12 (Reverse primer):
         GGTGTTGGCCTCCCGGGCCACTAGTTGAKGAGACRGTGACCTGGGT.
      SEQ ID NO: 13 (Reverse primer):
         GGTGTTGGCCTCCCGGGCCACTAGTTGAKGAGACRGTGACCAGGGT.
5. Amplify to obtain camel/alpaca VHH gene fragments.

The camel/alpaca VHH fragments obtained by the above amplification were recovered and linked to the pADL-23c (Biovector) bacteriophage vector by Sfi I enzyme digestion, and then electrotransformed to TG1 E. coli competent cells to establish an immune alpaca/camel VHH heavy chain antibody llibrary, wherein the capacity of NKp46 camel immune library was 5.2E7 and the capacity of alpaca immune library was 7.8E7.

### Example 4: Screening of positive clones that specifically bind to NKp46

In order to obtain positive antibodies that can cross-bind to human NKp46 and cynomolgus monkey NKp46, the above libraries were amplified and added into M13K07 helper phage and assembled into bacteriophages. 1×10¹² pfu of immune camel antibody library and immune alpaca antibody library were added thereto, and the phages were incubated with biotinylated human NKp46 protein (8 µg/mL) bound to magnetic beads for 1 h at room temperature. After washing off the unbound bacteriophages with 0.05% PBST, the phages specifically bound to NKp46 were eluted with 100nM triethylamine, and after gradient dilution of the bacteriophages above, E. coli SS320 in the logarithmic growth phase were infected. After being cultured overnight at 37°C on plates containing ampicillin, single-clone was picked for IPTG-induced expression, and the supernatant was used for ELISA assay. ELISA plates were coated with 2µg/mL human NKp46 antigen or cynomolgus monkey NKp46 antigen overnight at 4°C, then washed 3 times with 0.05% PBST and blocked with 5% skim milk at room temperature for 1h, and washed 3 times with 0.05% PBST. Then 30µL of induced supernatant was then added into per well, , medium were added to negative control wells, the ELISA plates were incubated at room temperature for 1h, and finally detected with anti-Myc HRP (VHH with his and c-Myc labels were expressed by the induction of IPTG. Sequence clones that had OD450 values of greater than 1.0 for binding to human NKp46 and cynomolgus monkey NKp46 as determined by ELISA, and of which the ratio of OD450 values to that of negative control were greater than 3 were sequenced, thereby obtaining amino acid sequences of 30 variable regions of heavy chain antibodies disclosed in the present disclosure, as shown in Table 2.

**Table 2. Amino acid sequences of 30 variable regions of heavy chain antibodies**

| Clone number | Variable region of heavy chain |
|---|---|
| NKp46-PC 1 | SEQ ID NO: 14 |
| NKp46-PC2 | SEQ ID NO: 15 |
| NKp46-PC3 | SEQ ID NO: 16 |
| NKp46-PC4 | SEQ ID NO: 17 |
| NKp46-PC6 | SEQ ID NO: 18 |
| NKp46-PC7 | SEQ ID NO: 19 |
| NKp46-PC8 | SEQ ID NO: 20 |
| NKp46-PC9 | SEQ ID NO: 21 |
| NKp46-PC 11 | SEQ ID NO: 22 |
| NKp46-PC12 | SEQ ID NO: 23 |
| NKp46-PC13 | SEQ ID NO: 24 |
| NKp46-PC16 | SEQ ID NO: 25 |
| NKp46-PA2 | SEQ ID NO: 26 |
| NKp46-PA3 | SEQ ID NO: 27 |
| NKp46-PA11 | SEQ ID NO: 28 |
| NKp46-PA15 | SEQ ID NO: 29 |
| NKp46-PA19 | SEQ ID NO: 30 |
| NKp46-PA22 | SEQ ID NO: 31 |
| NKp46-PA24 | SEQ ID NO: 32 |
| NKp46-PA26 | SEQ ID NO: 33 |
| NKp46-PA27 | SEQ ID NO: 34 |
| NKp46-PA28 | SEQ ID NO: 35 |
| NKp46-PA29 | SEQ ID NO: 36 |
| NKp46-PA31 | SEQ ID NO: 37 |
| NKp46-PA34 | SEQ ID NO: 38 |
| NKp46-PA36 | SEQ ID NO: 39 |
| NKp46-PA40 | SEQ ID NO: 40 |
| NKp46-PA45 | SEQ ID NO: 41 |
| NKp46-PA63 | SEQ ID NO: 42 |
| NKp46-PA69 | SEQ ID NO: 43 |

Based on the above amino acid sequences, the Kabat numbering scheme is used to divide the CDR and FR in the variable region of the antibody, and the 3 CDR sequences of each antibody are shown in Table 3 below. Numbers in parentheses in Table 3 indicate serial numbers, for example, (44) represents the SEQ ID NO: 44.

**Table 3. CDR sequences of 30 heavy chain antibodies**

| Clone number | HCDR1 | HCDR2 | HCDR3 |
|---|---|---|---|
| NKp46-PC 1 | TDCMG (44) | TIYTSDGRTDYANSVKG (45) | DPQYGGVCPSGGWNY (46) |
| NKp46-PC2 | NYNMF (47) | VINRGGDTADYAASVKG (48) | DPLGTT (49) |
| NKp46-PC3 | TMG (50) | CIYTGPTTTFTDYADSVK G (51) | NLAYPLTIIPDTR (52) |
| NKp46-PC4 | IEYMGW G (53) | YINTGGGTSVYDDSVKG (54) | GSFFGIWYKVPATQYFHY (55) |
| NKp46-PC6 | HYCMG (56) | LINTGGPTTFYADFVEG (57) | GPPSSDSGSGCYVPEYMYNY (58) |
| NKp46-PC7 | TDCMG (59) | TIYTSDGRTAYADSVKG (60) | DLQYGGSCPSGGWKY (61) |
| NKp46-PC8 | TGCMA (62) | IINISGTTRYTDSVKG (63) | TQNPRTVGRGYCTGDYFQVGGGGY SF (64) |
| NKp46-PC9 | RYCMA (65) | CLDRDGSTTYADSVKG (66) | AQPGDCWGRRYGFNT (67) |
| NKp46-PC 11 | SFSMA (68) | CIQAESGSTNVAPSVKG (69) | FKRNVGGCELRPEHWRF (70) |
| NKp46-PC 12 | SCGMD (71) | RIRPDGRTDYVESVKG (72) | WGLCTAKFR (73) |
| NKp46-PC 13 | IYYMG (74) | VMHTGGGSTYYTDSVK G (75) | SKYLLAFGGVEWILRPAGGWDY (76) |
| NKp46-PC 16 | IVSMS (77) | WINGDSSNTSYADSVKG (78) | QNSHYDIRFGY (79) |
| NKp46-PA2 | SYAMG (80) | AISRISGDTYSPDSVKG (81) | SATPSATNIYTNQYAYGD (82) |
| NKp46-PA3 | TIH (83) | RIWWIERTTFYAASVKG (84) | DARYTGSRRWESDY (85) |
| NKp46-PA11 | WYRMG (86) | RISGSPGILYYAGSVKG (87) | DRTGATWDY (88) |
| NKp46-PA15 | TYAMG (89) | ASSRDGTTTYYADSVKG (90) | SRPLSTTQVGIASAWYEY (91) |
| NKp46-PA19 | TYAMG (92) | AISRNGGTTYYADSVKG (93) | SRPLSNTQVGVASAWYEF (94) |
| NKp46-PA22 | TLH (95) | RIWWIGGATFYADSVKG (96) | DARYTSNRRWESDY (97) |
| NKp46-PA24 | IYGMG (98) | AINWSSGHTYYADSVKG (99) | DSIYGLSFTVKDYDY (100) |
| NKp46-PA26 | TIH (101) | RIWWIGGATFYADSVKG (102) | DARYTSHGYYESDY (103) |
| NKp46-PA27 | WYRMG (104) | RISGDTNIKYYAGSVKG (105) | DRTGATWDY (106) |
| NKp46-PA28 | TYAMG (107) | AISRNADTTYYADSVKG (108) | DRYSSNTQVGVARTYYDY (109) |
| NKp46-PA29 | IYGMG (110) | AINWNSGHTYYADSVKG (111) | DSIIGLSFTVKDYDY (112) |
| NKp46-PA31 | RLPMG (113) | AISWSSSTTYYADSVKG (114) | VGGSLDYSATVVYTAARAYAD (115) |
| NKp46-PA34 | FYRMG (116) | RISSSAGLIYYVDSVKG (117) | DRHGTRWDY (118) |
| NKp46-PA36 | RYAMG (119) | AISSSGDPTYYADSVKG (120) | GLTARDTTVVVHPNGYSY (121) |
| NKp46-PA40 | RYSMG (122) | AISSSGDVTHYADSAKG (123) | SLTARATTVTVHPNGYNY (124) |
| NKp46-PA45 | RLPMA (125) | AISWSGGSTYYADSMKG (126) | VGDSAPYSATIVYTDARAYAY (127) |
| NKp46-PA63 | SYAMG (128) | AINRSGDFPYYADSVKG (129) | APRAPATQVVISAFGYEY (130) |
| NKp46-PA69 | LYAMG (131) | GISRTGDTTYSPDSVKG (132) | SATYSATNIYTHQRAYGD (133) |

### Example 5: Construction of anti-NKp46 chimeric antibodies, and their transient transfection and expression in eukaryotic cells

Transfection-grade expression plasmids were prepared by introducing the interest gene fragment to a pTT5 expression vector (Nova lifetech), wherein the interest gene fragment was generated after splicing the previously disclosed monoclonal antibody variable regions with human IgG1 constant region as shown in SEQ ID NO: 134.

Expi293F^{™} cells (Thermo Fisher Scientific) were cultured in serum-free medium, seeded in a shaker flask (Corning) and cultured on a shaker at 37°C, 8% CO₂. The cell density was adjusted, and the pTT5 recombinant expression vector containing the interest gene fragment were mixed with PEI transfection reagent at appropriate ratios to obtain a mixture. The mixture was added to a shaker for cell culture, after culturing 6 days, the expression supernatant was collected, cell debris was removed by high-speed centrifugation, and the affinity purification was performed with a Protein A column. the column was rinsed with PBS until A280 readings are decreased to the baseline. The interest protein was eluted with an acidic eluate at pH3.0-pH3.5 and neutralized with 1 M Tris-HCl, pH 8.0-9.0. After the eluted sample was properly concentrated, the solution was changed to PBS for subpackaging. The purity of the chimeric antibodies finally purified was analyzed by SDS-PAGE and HPLC, and the concentration thereof was determined by A280 method.

### Example 6: Binding of anti-NKp46 chimeric antibodies to NKp46-expressing cells

The stable human NKp46-CHOK1 and cynomolgus monkey NKp46-CHOK1 cell lines prepared in Example 2 were cultured, and the cells were digested with 0.25% pancreatic enzyme (containing EDTA) for about 5 minutes, then the complete medium was added to stop the reaction. After centrifugation at 1,500 rpm for 5 minutes, the supernatant was discarded. After resuspending with PBS containing 1% BSA, the cells were counted. The cell density was adjusted to 1E6/mL, and the cells were seeded into corning-3799 96-well plates at 100 µL/well, and cultured overnight at 37°C, 8% CO₂. Then the 96-well plate was centrifuged at 1500 rpm for 5 minutes, the supernatant was discarded and the precipitate was set aside at 4°C for later use.

The 24 anti-NKp46 chimeric antibodies obtained in the above example were selected, and the anti-NKp46 chimeric antibody solution was prepared with PBS containing 1% BSA, wherein anti-NKp46 chimeric antibody solution has an initial concentration of 100nM, and be diluted 10-fold to obtain 7 gradients respectively: 10⁻⁴ nM, 10⁻³ nM, 10⁻² nM, 10⁻¹ nM, 10⁰ nM, 10¹ nM and 10² nM.

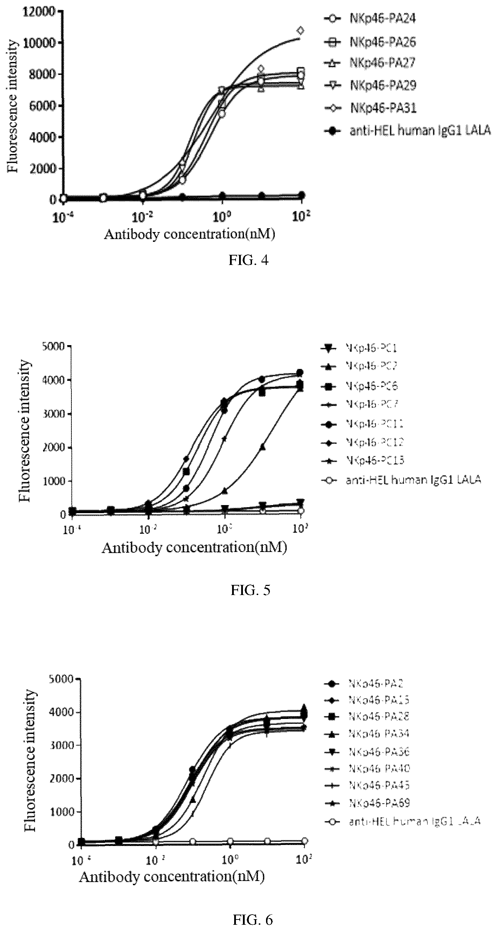

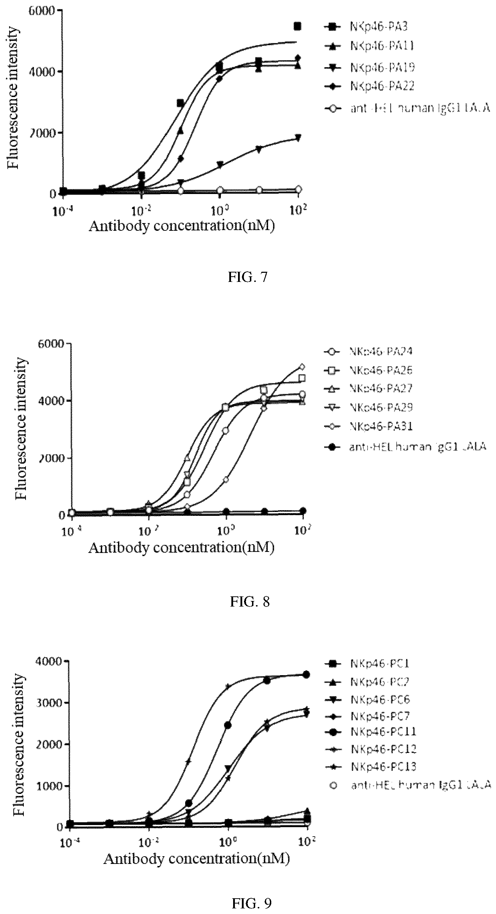

The cell culture on the plate was resuspended with the prepared antibody solution, 100 µL/well. The resuspended cell culture plates were incubated in a 4°C freezer for 1 h, and centrifuged at 1,500 rpm for 5 minutes, and the supernatant was discarded. After washing once with 160 µL of PBS containing 1% BSA, the supernatant was discarded, and the precipitate was stored for later use. The secondary antibody (goat anti human IgG Fc PE) solution was prepared with PBS containing 1% BSA, and diluted at a ratio of 1:200. Cells were resuspended with the prepared secondary antibody solution, 100 µL/well, incubated in a 4°C freezer for 0.5 h. After centrifuging at 1,500 rpm for 5 minutes, the supernatant was discarded, the precipitate was washed with 160 µL of PBS with 1% BSA, the supernatant was discarded, the cells were suspended with 100 µL of PBS containing 1% BSA, and filtered with a 300-mesh gauze. The mean fluorescence intensity of the antibody binding to cells was analyzed by a flow cytometer. The FCS file was exported from the flow cytometer, the PE channel mean fluorescence intensity (MFI) was analyzed with Flowjo software, and the analyzed mean fluorescence intensity was imported into Graphpad to analyze the concentration of antibodies and calculate the half-maximal binding to the cell (hereinafter referred to as EC50) and the top mean fluorescence intensity (Top MFI), as shown in Table 4 and Figures 3-10. All the anti-NKp46 chimeric antibodies bind well to cells overexpressing human and cynomolgus monkey NKp46.

**Table 4A. Binding of anti-NKP46 chimeric antibody to NKp46-expressing cells**

| Clone number | Human NKp46-CHOK1 | | Cynomolgus monkey NKp46-CHOK1 | |
|---|---|---|---|---|
| | EC₅₀ (nM) | Top mean fluorescence intensity (Top MFI) | EC₅₀ (nM) | Top mean fluorescence intensity (Top MFI) |
| Anti-HEL human IgG1 LALA | / | 223 | / | 118 |
| NKp46-PA3 | 0.149 | 9307 | 0.076 | 5445 |
| NKp46-PA11 | 0.162 | 7602 | 0.104 | 4178 |
| NKp46-PA19 | 0.09 | 7480 | 1.443 | 1788 |
| NKp46-PA22 | 0.365 | 7846 | 0.241 | 4413 |
| NKp46-PA24 | 0.495 | 7856 | 0.49 | 4190 |
| NKp46-PA26 | 0.396 | 8088 | 0.289 | 4747 |
| NKp46-PA27 | 0.155 | 7230 | 0.099 | 3935 |
| NKp46-PA29 | 0.2 | 7430 | 0.164 | 4018 |
| NKp46-PA31 | 0.535 | 10700 | 4.429 | 5140 |

**Table 4B. Binding of anti-NKp46 chimeric antibodies to NKp46-expressing cells**

| Clone number | Human NKp46-CHOK1 | | Cynomolgus monkey NKp46-CHOK1 | |
|---|---|---|---|---|
| | EC₅₀ (nM) | Top mean fluorescence intensity (Top MFI) | EC₅₀ (nM) | Top mean fluorescence intensity (Top MFI) |
| Anti-HEL human IgG1 LALA | / | 98 | / | 86 |
| NKp46-PC 1 | 7.052 | 310 | 9.813 | 180 |
| NKp46-PC2 | 18.82 | 3727 | 77.27 | 371 |
| NKp46-PC6 | 0.196 | 3828 | 1.06 | 2682 |
| NKp46-PC7 | 6.539 | 262 | 8.421 | 155 |
| NKp46-PC 11 | 0.416 | 4196 | 0.549 | 3646 |
| NKp46-PC12 | 0.138 | 3896 | 0.132 | 3669 |
| NKp46-PC13 | 0.911 | 4123 | 1.541 | 2808 |
| NKp46-PA2 | 0.075 | 3927 | 0.241 | 3414 |
| NKp46-PA15 | 0.102 | 3912 | 0.476 | 2549 |
| NKp46-PA28 | 0.093 | 3894 | 3.977 | 225 |
| NKp46-PA34 | 0.198 | 4122 | 0.169 | 3631 |
| NKp46-PA36 | 0.1 | 3757 | 0.483 | 2574 |
| NKp46-PA40 | 0.084 | 3516 | 26.83 | 453 |
| NKp46-PA45 | 0.237 | 3464 | 3.014 | 3105 |
| NKp46-PA69 | 0.096 | 3513 | 5.774 | 1502 |

### Example 7: Assay of in vitro NK cell activation by anti-NKp46 chimeric antibodies

Human fresh peripheral blood cells (PBMCs) were taken, then NK cells were isolated by using the EasySep^{™} Human NK Cell Isolation Kit (StemCell) and counted. Cells were cultured using α-MEM medium that was added by 15% heat-inactivated FBS, 0.2 mM inositol, 0.1 mM β-mercaptoethanol, 0.02 mM folic acid and 200 U/mL IL-2. The NK cell density was adjusted to 1E6/mL, and the cells were incubated in a 37°C, 5% CO₂ incubator for 6-8 days. The antibody was diluted with PBS at a maximum concentration of 20 nM, in 3-fold to obtain 7 gradients: 20 nM, 6.67 nM, 2.23 nM, 0.74 nM, 0.247 nM, 0.083 nM, and 0.027 nM, respectively. The diluted antibody solution was coated on a 96-well culture plate (Corning-3599) at 100 µL/well at 4°C overnight. The 96-well plate was washed twice using 150 µL/well PBS. The activated NK cell density was adjusted to 1E6/mL using complete medium, seeded into the washed 96-well plates, 100 µL/well, and cultured in a 37°C, 5% CO₂ incubator for 4 h. Cells were transferred from Corning-3599 plates to Corning-3799 plates. After centrifugation at 1,500 rpm for 5 minutes, the supernatant was discarded, and the precipitated cells were set aside for later use. CD3-APC (BD^{®}555335), CD56-BV421 (BD^{®}562751), CD107a-P (BD^{®}560948) were diluted as required by the instructions thereof, the cells obtained above were resuspended with those antibody solutions, in particular, the above CD3-APC, CD56-BV421, CD107a-PE antibody solutions were diluted at a concentration of 50 µL/well, and incubated at 4°C for 0.5 h. after centrifugation at 1,500 rpm for 5 minutes, the supernatant was discarded, the precipitate was washed with 160 µL of PBS containing 1% BSA, the supernatant was discarded, the cells were suspended with 100 µL of PBS containing 1% BSA, and filtered with a 300-mesh gauze. The percentage of CD107a-positive cells in NK was then analyzed by a flow cytometer. The FCS file was exported from the flow cytometer, the percentage of CD107a-positive cells in NK (CD3⁻CD56⁺) cells was analyzed with Flowjo software, and imported Graphpad to calculate the half effective concentration of antibodies that activate NK cells (hereinafter referred to as EC₅₀) and the top mean fluorescence intensity (Top MFI), as shown in Table 5 and Figures 11-12. Anti-NKp46 chimeric antibodies exhibit different activation capabilities on NK cell in vitro.

**Table 5A. In vitro NK cell activation by anti-NKP46 chimeric antibody**

| Clone number | EC₅₀ (nM) | Top mean fluorescence intensity (Top MFI) |
|---|---|---|
| Anti-HEL human IgG1 LALA | / | / |
| NKp46-PA11 | 2.20 | 16 |
| NKp46-PA24 | 0.31 | 15 |
| NKp46-PA27 | 1.93 | 20 |
| NKp46-PA31 | 0.08 | 23 |
| Anti-HEL human IgG1 LALA | / | / |

**Table 5B. In vitro NK cell activation of anti-NKP46 chimeric antibody**

| Clone number | EC₅₀ (nM) | Top mean fluorescence intensity (Top MFI) |
|---|---|---|
| Anti-HEL human IgG1 LALA | / | / |
| NKp46-PC6 | 0.51 | 30 |
| NKp46-PC11 | 0.54 | 26 |
| NKp46-PC 12 | 0.65 | 30 |
| NKp46-PA2 | 0.33 | 26 |
| NKp46-PA15 | 0.08 | 22 |
| NKp46-PA34 | 1.50 | 20 |
| NKp46-PA36 | 0.11 | 20 |
| NKp46-PA45 | 0.14 | 27 |
| NKp46-PA69 | 0.40 | 31 |

### Example 8: Humanization design of anti-NKP46 antibody

The above anti-NKP46 chimeric antibodies NKp46-PC11, NKp46-PA27, NKp46-PA31 and NKp46-PA45 were selected for humanization design.

### Example 8.1: Humanization of anti-NKP46-PC11

### (1) Selection of NKp46-PC11 human-derived framework

By sequence alignment, germline gene sequences with high homology to clone number NKp46-PC11 were selected as templates for VHH transplanted frameworks: IGHV3-23*04 (sequence identity 64.3%) and IGHJ4_01 (sequence identity 73.3%). After grafting the NKp46-PC11 CDR regions to the framework of variable region of the selected human antibody, the humanized antibody hPC11 was obtained, the humanized variable region hPC11 VHH-CDR graft sequence is shown in SEQ ID NO: 135:

EVQLVESGGGLVQPGGSLRLSCAASPYSANSFSMAWVRQAPGKGLEWVSCIQAESGSTNVAPSVK
GRFTISRDNSKNTLYLQMNSLRAEDTAVYYCAAFKRNVGGCELRPEHWRFWGQGTLVTVSS (SEQ

ID NO: 135), the sequence is FR1-CDR1-FR2-CDR2-FR3-CDR3-FR4, the sequences in italics are FR sequences, the underlined sequences are CDR sequences, graft represents the human variable region VHH sequence obtained after implantation of the CDR of chimeric antibodies into the germline FR region.

Revert mutations were made on hPC11 to obtain 5 humanized antibodies: H1-PC11 (the sequence is shown in SEQ ID NO: 136), H2-PC11 (the sequence is shown in SEQ ID NO: 137), H3-PC11 (the sequence is shown in SEQ ID NO: 138), H4-PC11 (the sequence is shown in SEQ ID NO: 139), and H5-PC11 (the sequence is shown in SEQ ID NO: 140).

### Example 8.2: Humanization of anti-NKP46-PA27

### (1) Selection of NKp46-PA27 human-derived framework

By sequence alignment, germline gene sequences with high homology to clone number NKp46-PA27 were selected as templates for VHH transplanted frameworks: IGHV3-30*15 (sequence identity 74.5%) and IGHJ4_01 (sequence identity 80%). After grafting the NKp46-PA27 CDR regions to the framework of variable region of the selected human antibody, the humanized antibody hPA27 was obtained, the humanized variable region hPa27 VHH-CDR graft sequence is shown in SEQ ID NO: 141: 
QVQLVESGGGVVQPGRSLRLSCAASGRTESWYRMGWVRQAPGKGLEWVARISGDTNIKYYAGSV
KGRFTISRDNSKNTLYLQMSSLRAEDTAVYYCAADRTGATWDYWGQGTLVTVSS (SEQ ID NO: 141),
 the sequences in italics are FR sequences, the underlined sequences are CDR sequences, graft represents the human variable region VHH sequence obtained after implantation of the CDRs of chimeric antibodies into the human germline FR region.

Revert mutations were made on hPA27 to obtain 4 humanized antibodies: H1-PA27 (the sequence is shown in SEQ ID NO: 141, the original grafted sequence), H2-PA27 (the sequence is shown in SEQ ID NO: 142), H3-PA27 (the sequence is shown in SEQ ID NO: 143), H4-PA27 (the sequence is shown in SEQ ID NO: 144).

### Example 8.3: Humanization of anti-NKP46-PA31

### (1) Selection of NKp46-PA31 human-derived framework

By sequence alignment, germline gene sequences with high homology to clone number NKp46-PA31 were selected as templates for VHH transplanted frameworks: IGHV3-23*04 (sequence identity 74.5%) and IGHJ4_01 (sequence identity 73.3%). After grafting the NKp46-PA31 CDR region to the framework of variable region of the selected human antibody, the humanized antibody hPA31 was obtained, the humanized variable region hPA31 VHH-CDR graft sequence is shown in SEQ ID NO: 145: 
EVQLVESGGGLVQPGGSLRLSCAASGRTFSRLPMGWVRQAPGKGLEWVSAISWSSSTTYYADSVK
GRFTISRDNSKNTLYLQMNSLRAEDTAVYYCAAVGGSLDYSATVVYTAARAYADWGQGTLVTVSS
 (SEQ ID NO: 145), wherein the sequences in italics are FR sequences, the underlined sequences are CDR sequences, graft represents the human variable region VHH sequence obtained after implantation of the CDRs of chimeric antibodies into the human germline FR region.

Revert mutations were made on hPA31 to obtain 4 humanized antibodies: H1-PA31 (the sequence is shown in SEQ ID NO: 145, the original grafted sequence), H2-PA31 (the sequence is shown in SEQ ID NO: 146), H3-PA31 (the sequence is shown in SEQ ID NO: 147), H4-PA31 (the sequence is shown in SEQ ID NO: 148).

### Example 8.4: Humanization of anti-NKP46-PA45

### (1) Selection of NKp46-PA45 human-derived framework

By sequence alignment, germline gene sequences with high homology to clone number NKp46-PA45 were selected as templates for VHH transplanted frameworks: IGHV3-23*04 (sequence identity 79.6%) and IGHJ4_01 (sequence identity 73.3%). After grafting the NKp46-PA31 CDR region to the framework of variable region of the selected human antibody, the humanized antibody hPA45 was obtained, the humanized variable region hPA45 VHH-CDR graft sequence is shown in SEQ ID NO: 149: 
EVQLVESGGGLVQPGGSLRLSCAASGRTFSRLPMAWVRQAPGKGLEWVSAISWSGGSTYYADSMK
GRFTISRDNSKNTLYLQMNSLRAEDTAVYYCAAVGDSAPYSATIVYTDARAYAYWGQGTLVTVSS
 (SEQ ID NO: 149), the sequences in italics are FR sequences, the underlined sequences are CDR sequences, graft represents the human variable region VHH sequence obtained after implantation of the CDR of chimeric antibodies into the germline FR region.

Revert mutations were made on hPA245 to obtain 4 humanized antibodies: H1-PA45 (the sequence is shown in SEQ ID NO: 149, the original grafted sequence), H2-PA31 (the sequence is shown in SEQ ID NO: 150), H3-PA31 (the sequence is shown in SEQ ID NO: 151), H4-PA31 (the sequence is shown in SEQ ID NO: 152).

### Example 9: Preparation of humanized antibody specifically bound to NKp46

By standard methods known to those skilled in the art, the fragment of gene of interest was generated after splicing the variable region of the humanized antibody and the constant region of human IgG1, and subcloned into a pTT5 expression vector (Nova lifetech). ExpiFectamine^{™} 293 transfection reagent was used to transfect Expi293F^{™} cells in the logarithmic growth phase, and the culture supernatant was collected and purified with affinity. The final purified humanized antibody was measured by SDS-PAGE and HPLC purity analysis and A280 concentration determination.

### Example 10: In vitro validation of cell binding to humanized antibody specifically binding to NKp46

The stable human NKp46-CHOK1 and cynomolgus monkey NKp46-CHOK1 cell lines obtained in Example 2 were cultured, the cells were digested with 0.25% pancreatic enzyme EDTA for about 5 minutes, and the digestion was terminated with the complete medium. After centrifugation at 1,500 rpm for 5 minutes, the supernatant was discarded, the precipitated cells were resuspended with PBS containing 1% BSA and counted. The cell density was adjusted to 1E6/mL and seeded in a corning-3799 96-well culture plate at 100 µL/well. Aftering centrifuging at 1,500 rpm for 5 minutes, the supernatant was discarded and the precipitate was set aside at 4 °C. The antibody solutions were prepared with PBS containing 1% BSA, wherein the antibody solution has an initial concentration was 100 nM, then diluted in 10-fold to obtain 7 gradients: 10⁻⁴ nM, 10⁻³ nM, 10⁻² nM, 10⁻¹ nM, 100 nM, 10¹ nM, and 10² nM. The prepared antibody solutions were used to resuspend the cells, 100 µL/well. The resuspended cell culture plates were incubated in a 4 °C freezer for 1 h. After centrifugation at 1500 rpm for 5 minutes, the supernatant was discarded. The precipitate was washed with 160 µL of PBS containing 1% BSA, the supernatant was discarded, and the cells were stored for later use.

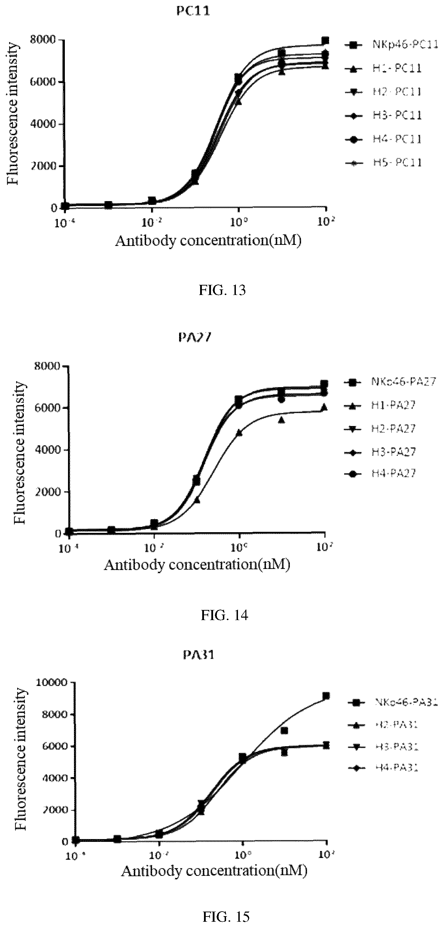

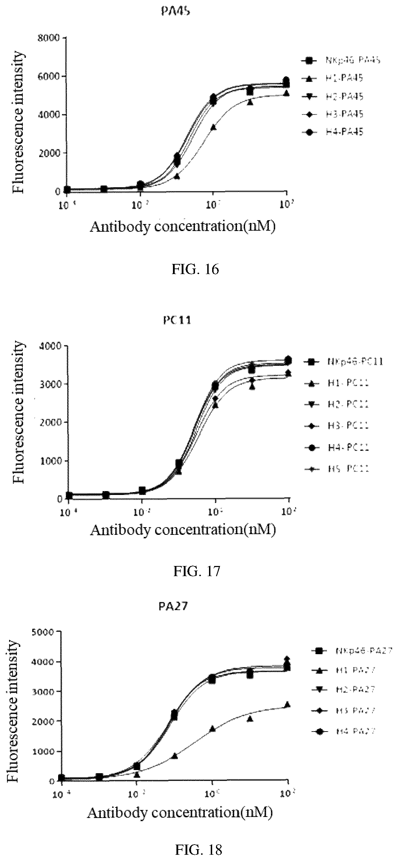

The Secondary antibody (goat anti human IgG Fc PE) solution was prepared with PBS containing 1% BSA, and diluted 1:200. The cells were resuspended with the prepared secondary antibody solution, 100 µL/well, incubated in a 4 °C freezer for 0.5 h. After centrifugation at 1,500 rpm for 5 minutes, the supernatant was discarded, the precipitated cells were washed with 160 µL of PBS containing 1% BSA, the supernatant was discarded, the cells were resuspended with 100 µL of PBS containing 1% BSA, and filtered in 300-mesh gauze. The mean fluorescence intensity of antibody binding to cells was then analyzed on a flow cytometer. The FCS file was exported from the flow cytometer, the mean fluorescence intensity (hereinafter referred to as MFI) of PE channel was analyzed with Flowjo software, and the mean fluorescence intensity obtained was introduced into the Graphpad to analyze the half binding concentration (hereinafter referred to as EC₅₀) and the top mean fluorescence intensity (Top MFI) of the antibody and the cell. The results were shown in Table 6 and Figure 13-20. Compared with the corresponding chimeric antibodies, the anti-NKp46 humanized antibody has comparable ability of binding to those cells overexpressing human NKp46 and cynomolgus monkey NKp46.

**Table 6. Binding of anti-NKp46 humanized antibodies to NKp46-expressing cells**

| Clone number | Human NKp46-CHOK1 | | Cynomolgus monkey NKp46-CHOK1 | |
|---|---|---|---|---|
| | EC₅₀ (nM) | Top mean fluorescence intensity (Top MFI) | EC₅₀ (nM) | Top mean fluorescence intensity (Top MFI) |
| NKp46-PC11 | 0.318 | 7887 | 0.261 | 3587 |
| H1-PC11 | 0.389 | 6710 | 0.345 | 3255 |
| H2-PC 11 | 0.363 | 6936 | 0.301 | 3535 |
| H3-PC11 | 0.328 | 6833 | 0.297 | 3285 |
| H4-PC 11 | 0.305 | 7240 | 0.274 | 3617 |
| H5-PC11 | 0.302 | 7370 | 0.270 | 3656 |
| NKp46-PA27 | 0.170 | 7094 | 0.077 | 3761 |
| H1-PA27 | 0.253 | 5995 | 0.338 | 2538 |
| H2-PA27 | 0.153 | 6938 | 0.069 | 3797 |
| H3-PA27 | 0.153 | 6818 | 0.072 | 4055 |
| H4-PA27 | 0.145 | 6646 | 0.074 | 3879 |
| NKp46-PA31 | 0.932 | 9082 | 3.708 | 4732 |
| H2-PA31 | 0.203 | 5938 | 2.107 | 3269 |
| H3-PA31 | 0.148 | 5979 | 1.157 | 3214 |
| H4-PA31 | 0.162 | 5954 | 1.301 | 3337 |
| NKp46-PA45 | 0.226 | 5517 | 2.064 | 3287 |
| H1-PA45 | 0.543 | 5093 | 11.11 | 3117 |
| H2-PA45 | 0.274 | 5613 | 2.762 | 3591 |
| H3-PA45 | 0.188 | 5720 | 1.516 | 3328 |
| H4-PA45 | 0.205 | 5762 | 1.533 | 3395 |

### Example 11: In vitro NK cell activation assay by anti-NKp46 humanized antibody

Human fresh peripheral blood cells (PBMCs) were taken, then NK cells were isolated by using the EasySep^{™} Human NK Cell Isolation Kit and counted. Cells were cultured using α-MEM medium that was added by 15% heat-inactivated FBS, 0.2 mM inositol, 0.1 mM β-mercaptoethanol, 0.02 mM folic acid, and 200 U/mL IL-2. The NK cell density was adjusted to 1E6/mL and incubated in a 37°C, 5% CO₂ incubator for 6-8 days. The antibody was diluted with PBS at a maximum concentration of 20 nM, in 3-fold, to obtain 7 gradients: 20 nM, 6.67 nM, 2.23 nM, 0.74 nM, 0.247 nM, 0.083 nM, and 0.027 nM, respectively. The diluted antibody solutions were coated on a 96-well culture plate at Corning-3599 at 100 µL/well at 4°C overnight. The 96-well plate was washed twice using 150 µL/well PBS. The activated NK cell density was adjusted to 1E6/mL using complete medium and seeded into washed a 96-well plate, 100 µL/well. The activated NK cells were cultured in a 37°C, 5% CO2 incubator for 4 h. The cells were transferred from Corning-3599 plates to Corning-3799 plates. After centrifugation at 1,500 rpm for 5 minutes, the supernatant was discarded, and the precipitated cells were set aside for later use.

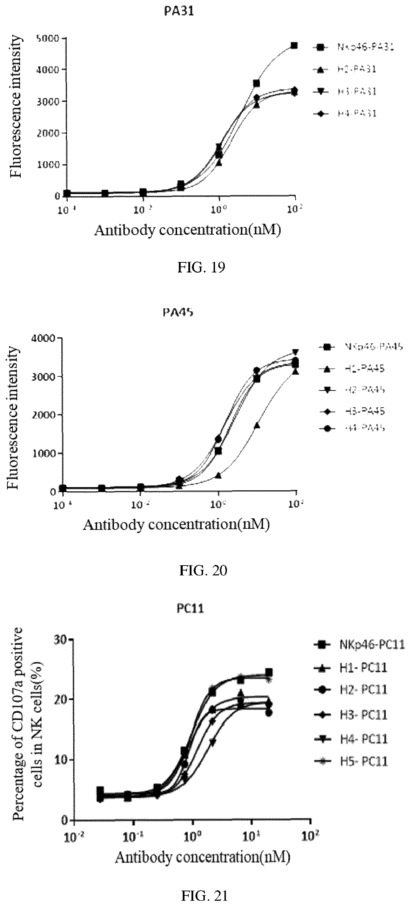

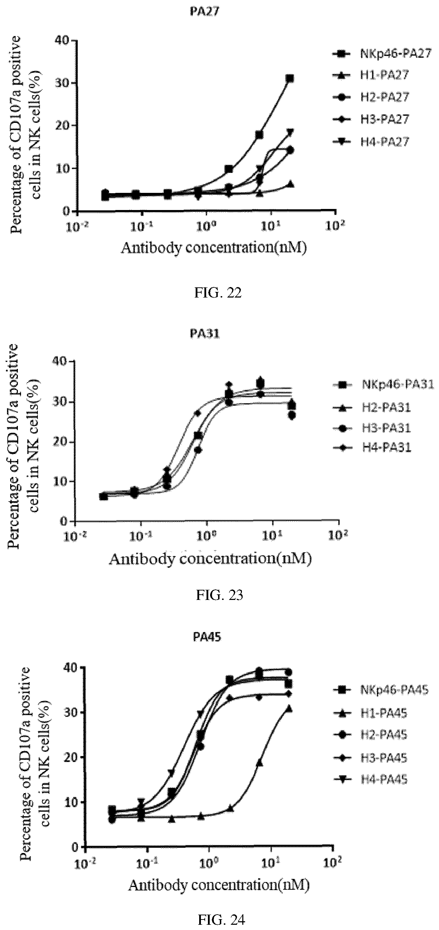

The antibodies (CD3-APC, CD56-BV421, CD107a-P) were diluted according to the instructions. The cells obtained above were suspended, 50 µL/well, and incubated at 4°C for 0.5 h. After centrifuging at 1,500 rpm for 5 minutes, the supernatant was discarded, the precipitate was washed with 160 µL of PBS containing 1% BSA, the supernatant was discarded. After washing with 100 µL of PBS containing 1% BSA, cells were washed in a 300-mesh gauze. The percentage of CD107a-positive cells in NK cells is then analyzed on a flow cytometer. The FCS file were exported from the flow cytometer, and the percentage of CD107a-positive cells in NK (CD3⁻CD56⁺) cells was analyzed with Flowjo software, and imported Graphpad to find the half effective concentration of NK activation (hereinafter referred to as EC₅₀) and the top mean fluorescence intensity (Top MFI), as shown in Table 7 and Figures 21-24. Compared with the anti-NKp46 humanized antibody and the corresponding chimeric antibody, the activation ability of NK cells in vitro maintained or slightly weakened.

**Table 7 In vitro NK cell activation by anti-NKP46 chimeric antibody**

| Clone number | EC₅₀ (nM) | Top mean fluorescence intensity (Top MFI) |
|---|---|---|
| NKp46-PC11 | 0.97 | 24.4 |
| H1-PC11 | 0.85 | 21.0 |
| H2-PC 11 | 0.87 | 18.8 |
| H3-PC11 | 1.21 | 19.6 |
| H4-PC 11 | 1.84 | 19.3 |
| H5-PC11 | 0.95 | 23.6 |
| NKp46-PA27 | 15.92 | 30.9 |
| H1-PA27 | >20 | 6.3 |
| H2-PA27 | >20 | 14.1 |
| H3-PA27 | 7.39 | 14.4 |
| H4-PA27 | 10.73 | 18.1 |
| NKp46-PA31 | 0.62 | 34.4 |
| H2-PA31 | 0.63 | 35.3 |
| H3-PA31 | 0.75 | 31.6 |
| H4-PA31 | 0.38 | 34.1 |
| NKp46-PA45 | 0.63 | 38.1 |
| H1-PA45 | 7.39 | 30.8 |
| H2-PA45 | 0.75 | 39.1 |
| H3-PA45 | 0.58 | 34.0 |
| H4-PA45 | 0.40 | 37.6 |

### Example 12: Biacore affinity experiment that specifically binds to NKp46 humanized antibodies

The Biacore 8K instrument was used to analyze the affinity and kinetic properties of NKp46 humanized antibodies that specifically bind to human NKp46 (Kactus, NKP-HM146) and cynomolgus monkey NKp46 (Kactus, NKP-CM146). The CM5 chip of Biacore instrument was first activated with ethyl-dimethylaminopropyl-carbodiimide EDC and hydroxysuccinimide NHS, then immobilized by the mouse antibody against human Fc, and blocked with ethanolamine.

To determine the affinity and kinetic properties with human NKp46, the NKp46 humanized antibody was diluted to 0.8 µg/mL with HBS-EP+ buffer (10 mM HEPES, pH 7.4, 150 mM NaCl, 3 mM EDTA, 0.05% P20) and captured at a flow rate of 10 µL/min for 60 s. Human NKp46 was diluted in two-fold to a serial concentration (400 nM-3.125 nM), then combined for 90 s at a flow rate of 50 µL/min, and dissociated for 500 s.

To determine the affinity and kinetic properties with cynomolgus monkey NKp46, the NKp46 humanized antibody was diluted to 0.8 µg/mL with HBS-EP+ buffer (10 mM HEPES, pH 7.4, 150 mM NaCl, 3 mM EDTA, 0.05% P20) and captured at a flow rate of 10 µL/min for 60 s. Cynomolgus monkey NKp46 was diluted in two-fold to a serial concentration (400 nM-3.125 nM), combined for 90 s at a flow rate of 50 µL/min and dissociated for 500 s.

At the end of each round of experiments, 3 M MgCh solution was used to rinse at a flow rate of 30 µL/min for 30 s, and the captured antibody was removed along with the antigen to complete the regeneration of the chip. The raw data were analyzed using Biacore Insight Evaluation Software (version 3.0.12.15655) software and (1:1) Langmuir model was fit to the data.The results are shown in Table 8. Compared with the anti-NKp46 humanized antibody and the corresponding chimeric antibody, the affinity to the human NKp46 antigen protein is mostly maintained.

**Table 8A. Results of affinity assay of anti-NKp46 humanized antibody to human NKp46 antigen protein**

| Clone number | ka (1/Ms) | kd(1/s) | KD(M) | Rmax (RU) |
|---|---|---|---|---|
| NKp46-PC 11 | 7.02E+04 | 3.40E-04 | 4.84E-09 | 72.4 |
| H1-PC11 | 5.08E+04 | 3.07E-04 | 6.05E-09 | 93.9 |
| H2-PC 11 | 6.13E+04 | 1.26E-04 | 2.05E-09 | 55.1 |
| H3-PC11 | 6.78E+04 | 7.75E-05 | 1.14E-09 | 50 |
| NKp46-PA27 | 1.12E+05 | 3.62E-03 | 3.25E-08 | 94.7 |
| H2-PA27 | 9.77E+04 | 2.16E-03 | 2.21E-08 | 119.3 |
| H4-PA27 | 1.12E+05 | 2.66E-03 | 2.37E-08 | 117.1 |
| NKp46-PA31 | 1.68E+05 | 1.00E-03 | 5.97E-09 | 77.3 |
| H2-PA31 | 8.78E+04 | 9.59E-04 | 1.09E-08 | 45.4 |
| H4-PA31 | 1.75E+05 | 1.03E-03 | 5.89E-09 | 46.6 |
| NKp46-PA45 | 8.59E+04 | 1.87E-03 | 2.18E-08 | 48.2 |
| H2-PA45 | 6.38E+04 | 1.54E-03 | 2.41E-08 | 59.7 |
| H4-PA45 | 9.30E+04 | 2.04E-03 | 2.19E-08 | 68.2 |

**Table 8B. Results of affinity assay of anti-NKp46 humanized antibody to monkey NKp46 antigen protein**

| Clone number | ka (1/Ms) | kd(1/s) | KD(M) | Rmax (RU) |
|---|---|---|---|---|
| H1-PC11 | 5.13E+04 | 5.89E-03 | 1.15E-07 | 102.1 |
| H2-PA27 | 2.52E+05 | 5.90E-03 | 2.34E-08 | 171.7 |
| H2-PA31 | 7.75E+04 | 5.96E-04 | 7.69E-09 | 44.8 |
| H2-PA45 | 7.67E+04 | 5.63E-04 | 7.34E-09 | 39.5 |

### Example 13: Test of physical stability of anti-NKp46 humanized antibodies

NanoDSF (differential fluorescence scanning technology) was used to detect the thermal stability of different antibodies in pH 7.4 PBS buffer. The sample concentration was about 1 mg/m, and tested by using Prometheus NT. Plex instruments. each sample was centrifuged at 10,000 g for 10 minutes before testing. 40 µL of sample was added to each well in the sample plate (the amount of loading on the instrument is 10 µL, and each sample was performed in double). The temperature starts at 30°C and ends at 95°C, at a scanning rate of 0.5°C/min. The experimental results are shown in Table 9.

**Table 9. Results of NanoDSF test results for anti-NKp46 humanized antibody**

| Clone number | Tm Onset | Tm1 | Tm2 | Tm3 | Tagg |
|---|---|---|---|---|---|
| NKp46-PC 11 | 62.6°C | 68.5°C | 76.9°C | / | 76.5°C |
| H1-PC11 | 61.2°C | 69.0°C | 79.4°C | / | 77.3°C |
| H2-PC 11 | 61.1°C | 68.4°C | 77.2°C | / | 76.4°C |
| H3-PC11 | 62.5°C | 67.6°C | 75.6°C | / | 76.8°C |
| H4-PC 11 | 62.0°C | 65.8°C | 74.8°C | / | 76.0°C |
| H5-PC11 | 62.0°C | 67.3°C | 75.8°C | / | 77.0°C |
| NKp46-PA27 | 62.4°C | 67.3°C | 73.6°C | 80.3°C | 70.1°C |
| H2-PA27 | 58.9°C | 62.5°C | / | / | 62.1°C |
| H3-PA27 | 57.6°C | 60.9 °C | 79.9°C | / | 60.1°C |
| H4-PA27 | 58.9°C | 61.4°C | 80.7°C | / | 61.2°C |
| NKp46-PA31 | 62.7 °C | 64.2°C | 80.0°C | / | 64.9 °C |
| H2-PA31 | 60.7 °C | 67.9°C | 80.3°C | / | 69.0°C |
| H3-PA31 | / | 64.9 °C | 80.4°C | / | 65.8°C |
| H4-PA31 | 73.9°C | 80.1°C | / | / | 66.3°C |
| NKp46-PA45 | 65.3°C | 67.4°C | 80.4°C | / | 67.9°C |
| H2-PA45 | 62.1°C | 69.5°C | 76.8°C | 81.3°C | 75.6°C |
| H3-PA45 | 63.6°C | 66.3°C | 73.1°C | 81.0°C | 72.1°C |
| H4-PA45 | 63.1°C | 67.0°C | 73.9°C | 81.3°C | 72.9°C |

SEC-HPLC was used to monitor the sample purity and investigate the periodic stability at certain concentrations. Regarding exemplary conditions, the sample concentrations were controlled in 10 mg/mL, 10 mM acetate, 9% trehalose (pH 5.5) buffer, and the concentrations were controlled in 1 mg/mL PBS (PH 7.4), the stability of samples that stored for 0, 7, and 14 days at 40 °C were compared. The Waters Xbridge Protein BEH SEC 3.5µm 7.8*300mm column was used to detect the samples, wherein the mobile phase is PBS buffer which is adjusted to pH 6.8 using H₃PO4, and the flow rate was 0.5 mL/min. The experimental results are shown in Table 10.

**Table 10. Results of cyclic stability of anti-NKp46 humanized antibody (SEC-HPLC)**

| | H1-PC11 | | H2-PA27 | | H2-PA31 | | H2-PA45 | |
|---|---|---|---|---|---|---|---|---|
| | pH5.5 | pH7.4 | pH5.5 | pH7.4 | pH5.5 | pH7.4 | pH5.5 | pH7.4 |
| 0 day | 98.81% | 98.93% | 99.43% | 99.52% | 97.81% | 97.66% | 98.65% | 98.72% |
| 7 days | 98.69% | 98.90% | 99.35% | 99.47% | 97.78% | 97.63% | 98.23% | 98.62% |
| 14 days | 98.50% | 98.32% | 99.51% | 98.77% | 97.64% | 97.32% | 98.36% | 98.38% |
| Variable value | -0.31% | -0.61% | +0.08% | -0.75% | -0.17% | -0.34% | -0.29% | -0.34% |

The molecular integrity of the samples was monitored by CE-SDS, and the periodic stability under certain concentration conditions was investigated. The sample concentration was controlled in 10 mg/mL, 10 mM acetate, 9% trehalose (pH 5.5) buffer, and the molecular integrity thereof were compared where the samples were stored at 40 °C for 0, 7, and 14 days under reducing and non-reducing conditions. 200 µg of sample (100 µg reduction, 100 µg non-reduction) was taken, sample buffer was added to 97 µL, 5 µL of 2-mercaptoethanol was added to the reduced samples, and 5 µL of 250 mM iodoacetamide was added to the non-reducing samples. All the samples were heated at 70 °C for 10 minutes. PA 800 Plus (Beckman Sciex) was used for detection, wherein the electrophoresis time is 40 minutes. The experimental results are shown in Table 11.

**Table 11. Results of cyclic stability of anti-NKp46 humanized antibody (CE-SDS)**

| | H1-PC11 | | H2-PA27 | | H2-PA31 | | H2-PA45 | |
|---|---|---|---|---|---|---|---|---|
| | non-r educt ion | Reduction | non-redu ctio n | Reduction | non-redu ctio n | Reduction | non -red ucti on | Reduction |
| 0 day | 100 % | 99.78% | 99.5 1% | 99.59% | 100 % | 99.42% | 98.8 0% | 99.64% |
| 7 days | 98.6 8% | 99.48% | 99.3 1% | 99.83% | 98.8 8% | 99.49% | 98.7 4% | 99.62% |
| 14 days | 98.6 2% | 99.53% | 97.6 4% | 99.66% | 97.6 6% | 99.43% | 97.6 5% | 99.50% |
| Variable value | -1.38 % | -0.25% | -1.8 7% | +0.07% | -2.3 4% | +0.01% | -1.1 5% | -0.14% |

### Example 14: Chemical stability test for anti-NKp46 humanized antibodies

The charge heterogeneity of sample molecules was monitored by cIEF. Antibodies have many post-translational modifications including deaminization, isomerization, terminal changes, glycosylation, oxidation, cleavage, polymerization, etc. These modifications may cause changes in the charges on the surface of the antibodies, resulting in charge heterogeneity. Based on the charges of the antibodies, cIEF may separate them, and then analyze the charge heterogeneity of the antibodies. The sample concentration was controlled in 10 mg/mL, 10 mM acetate, 9% trehalose (pH 5.5) buffer, and the charge heterogeneity of samples that stored for 0, 7, and 14 days at 40 °C were compared. 20 µg sample was taken, and Separation Mix buffer was added to the samples. The antibody samples were focused for 7 minutes by using Maurice.C (ProteinSimple) , and the charge distribution thereof were detected. The experimental results are shown in Table 12.

**Table 12. Results of cyclic stability of anti-NKp46 humanized antibody (cIEF)**

| | H1-PC11 | H2-PA27 | H2-PA31 | H2-PA45 |
|---|---|---|---|---|
| Acidic peak changes | + 11.5% | + 11.4% | + 7.7% | + 9.7% |
| Principal peak changes | -16.3% | -12.5% | - 20.5% | - 15.3% |
| Alkaline peak changes | + 4.7% | + 1% | + 12.8% | + 5.6% |

Post-translational modifications of antibody samples were detected by LC-MS technique. Deaminization modification is a common chemical modification in antibodies that may affect the stability at later stages, especially highly deaminization modifications on parts of amino acids in CDR regions are generally avoided as much as possible, or mutations are reduced. Exemplary conditions include: the concentration of the antibodies to be tested was controlled in 10 mg/mL, 10 mM acetate, 9% trehalose (pH 5.5) buffer, and the concentration was controlled in 1 mg/mL PBS (pH 7.4) buffer. The samples were stored in a 40°C incubator. Samples were taken on days 0, 7 and 14 for enzymatic digestion experiments. 30 µg of antibody samples were taken and 6 µL of 8 M guanidine hydrochloride and 3 µL of 200 mM DTT were added thereto. After a 60 °C water bath for 20 minutes, 46 µL of 20 mM Histinine buffer (pH 6.0) and 2 µL of PNGase F enzyme (Promega, V5111) were added thereto, and the samples were placed in a 37 °C water bath for 16 h. On the Q-Exactive Plus instrument, LC-MS is performed to detect deaminization modifications. The experimental results are shown in Table 13.

**Table 13. Results of cyclic stability of anti-NKp46 humanized antibody chemical modifications**

| H1-PC11 | Chemical modification type | pH5.5 | | pH7.4 | |
|---|---|---|---|---|---|
| | | starting value on day 0 | change value during 14 days | starting value on day 0 | change value during 14 days |
| | M34 Oxidation, Methionine oxidation at position 34 | 10.06% | -2.25% | 4.95% | -0.05% |
| | N59 Loss N, Asparagine denitrification at position 59 | 7.91% | 0.59% | 22.34% | -0.94% |
| | M83 Oxidation, Methionine oxidation at position 83 | 10.94% | -2.64% | 9.91% | -1.01% |
| H2-PA27 | M34 Oxidation, Methionine oxidation at position 34 | 8.02% | 1.22% | 10.09% | -0.45% |
| H2-PA31 | M34 Oxidation, Methionine oxidation at position 34 | 9.72% | -0.02% | 9.29% | 0.77% |
| H2-PA45 | No significant chemical modification changes were detected | / | / | / | / |

Note: N represents the modified asparagine detected, M represents the modified methionine detected, the number represents the serial number of the position that counts from the N-terminal end of the light or heavy chains. The percentage represents the percentage of deamidation modifications detected by LC-MS to the total signal peptide at that site.

From the above experimental data, it can be seen that the four humanized antibodies such as H1-PC11, H2-PA27, H2-PA31 and H2-PA45 exhibit high heat deflection temperatures, and after 14 days of storage at a high temperature of 40°C, the change of the percentages of polymer and fragment is smaller, and the changes of the percentages of chemical modifications is lower, thus both the physical stability and chemical stability of these four humanized antibodies are good.

The embodiments of the present disclosure described above are intended to be merely exemplary, and equivalents of numerous specific compounds, materials, and operations may be recognized or determined by those skilled in the art without unconventional experiments, such equivalents are intended to be within the scope of the present disclosure and are encompassed by the claims.

## Claims

1. An anti-NKp46 antibody or antigen-binding fragment thereof being capable of specifically binding to NKp46, comprising 3 CDRs selected from sequences shown in SEQ ID NOs: 44-133 sequence.

2. An anti-NKp46 antibody or antigen-binding fragment thereof being capable of specifically binding to NKp46, comprising a heavy chain variable region, wherein the heavy chain variable region comprises HCDR1 selected from a group consisting of SEQ ID NOs: 44, 47, 50, 53, 56, 59, 62, 65, 68, 71, 74, 77, 80, 83, 86, 89, 92, 95, 98, 101, 104, 107, 110, 113, 116, 119, 122, 125, 128, 131; and HCDR2 selected from a group consisting of SEQ ID NOs: 45, 48, 51, 54, 57, 60, 63, 66, 69, 72, 75, 78, 81, 84, 87, 90, 93, 96, 99, 102, 105, 108, 111, 114, 117, 120, 123, 126, 129, 132; and HCDR3 selected from a group consisting of SEQ ID NOs: 46, 49, 52, 55, 58, 61, 64, 67, 70, 73, 76, 79, 82, 85, 88, 91, 94, 97, 100, 103, 106, 109, 112, 115, 118, 121, 124, 127, 130, 133;
preferably, the heavy chain variable region comprises HCDR1, HCDR2, HCDR3 selected from the following groups: SEQ ID NO: 44, SEQ ID NO: 45 and SEQ ID NO: 46; or SEQ ID NO: 47, SEQ ID NO: 48 and SEQ ID NO: 49; or SEQ ID NO: 50, SEQ ID NO: 51 and SEQ ID NO: 52; or SEQ ID NO: 53, SEQ ID NO: 54 and SEQ ID NO: 55; or SEQ ID NO: 56, SEQ ID NO: 57 and SEQ ID NO: 58; or SEQ ID NO: 59, SEQ ID NO: 60 and SEQ ID NO: 61; or SEQ ID NO: 62, SEQ ID NO: 63 and SEQ ID NO: 64; or SEQ ID NO: 65, SEQ ID NO: 66 and SEQ ID NO: 67; or SEQ ID NO: 68, SEQ ID NO: 69 and SEQ ID NO: 70; or SEQ ID NO: 71,SEQ ID NO: 72 and SEQ ID NO: 73; or SEQ ID NO: 74, SEQ ID NO: 75 and SEQ ID NO: 76; or SEQ ID NO: 77, SEQ ID NO: 78 and SEQ ID NO: 79; or SEQ ID NO: 80, SEQ ID NO: 81 and SEQ ID NO: 82; or SEQ ID NO: 83, SEQ ID NO: 84 and SEQ ID NO: 85; or SEQ ID NO: 86, SEQ ID NO: 87 and SEQ ID NO: 88; or SEQ ID NO: 89, SEQ ID NO: 90 and SEQ ID NO: 91; or SEQ ID NO: 92, SEQ ID NO: 93 and SEQ ID NO: 94; or SEQ ID NO: 95, SEQ ID NO: 96 and SEQ ID NO: 97; or SEQ ID NO: 98, SEQ ID NO: 99 and SEQ ID NO: 100; or SEQ ID NO: 101, SEQ ID NO: 102 and SEQ ID NO: 103; or SEQ ID NO: 104, SEQ ID NO: 105 and SEQ ID NO: 106; or SEQ ID NO: 107, SEQ ID NO: 108 and SEQ ID NO: 109; or SEQ ID NO: 110, SEQ ID NO: 111 and SEQ ID NO: 112; or SEQ ID NO: 113, SEQ ID NO: 114 and SEQ ID NO: 115; or SEQ ID NO: 116, SEQ ID NO: 117 and SEQ ID NO: 118; or SEQ ID NO: 119, SEQ ID NO: 120 and SEQ ID NO: 121; or SEQ ID NO: 122, SEQ ID NO: 123 and SEQ ID NO: 124; or SEQ ID NO: 125, SEQ ID NO: 126 and SEQ ID NO: 127; or SEQ ID NO: 128, SEQ ID NO: 129 and SEQ ID NO: 130; or SEQ ID NO: 131, SEQ ID NO: 132 and SEQ ID NO: 133.

3. An anti-NKp46 antibody or antigen-binding fragment thereof being capable of specifically binding to NKp46, comprising HCDR1, HCDR2 and HCDR3 selected from a heavy chain variable region as shown in SEQ ID NOs: 14-43, 136-152;
preferably, the heavy chain variable region has at least 80% to 100% sequence identity to the sequences as shown in SEQ ID NOs: 14-43, 136-152.

4. The anti-NKp46 antibody or antigen-binding fragment thereof according to any of claims 1 to 3, wherein the antibody is a recombinant antibody, preferably, the antibody is an alpaca-derived antibody, a chimeric antibody or a humanized antibody; more preferably, the antibody is a nanobody; further preferably, the antibody is a humanized camelid VHH.

5. The anti-NKp46 antibody or antigen-binding fragment thereof according to any of the preceding claims, further comprising a heavy chain constant region and/or light chain constant region, preferably, the heavy chain constant region comprises Fc or variant Fc, the Fc is derived from mice or humans; and/or,
the anti-NKp46 antibody or antigen-binding fragment thereof is in the formats of IgG1, IgG2, IgG3, or IgG4.

6. A conjugate formed by coupling the anti-NKp46 antibody or antigen-binding fragment thereof according to any of the preceding claims to a capture label or a detection label, wherein the detection label comprises radionuclides, luminescent substances, colored substances, or enzymes.

7. A bispecific antibody or multi-specific antibody, wherein an antigen-binding domain comprises the anti-NKp46 antibody or antigen-binding fragment thereof according to any of the preceding claims.

8. An antibody-drug conjugate, comprising the anti-NKp46 antibody or antigen-binding fragment thereof according to any of the preceding claims, the antibody-drug conjugate is formed by antibody-linker-toxin interconnections.

9. A chimeric antigen receptor, wherein the extracellular recognition unit comprises the anti-NKp46 antibody or antigen-binding fragment thereof according to any of the preceding claims.

10. A nucleic acid encoding the anti-NKp46 antibody or antigen-binding fragment thereof according to any of the preceding claims; or a recombinant vector comprising the nucleic acid; or a host cell comprising the recombinant vector or an integrated genome comprising the nucleic acid.

11. A method of preparing the anti-NKp46 antibody or antigen-binding fragment thereof according to any of the preceding claims, comprising:
culturing the host cells according to claim 10 under suitable conditions and purifying the expression products from the cells.

12. Use of the anti-NKp46 antibody or antigen-binding fragment thereof according to any of the preceding claims in the preparation of drugs for specifically targeting NKp46-expressing cells; use of the anti-NKp46 antibody or antigen-binding fragment thereof according to any of the preceding claims in the treatment, prevention or detection of diseases that specifically express NKp46; preferably, the cells are NK cells.

13. Use of the anti-NKp46 antibody or antigen-binding fragment thereof according to any of the preceding claims in the preparation of drugs for cancers, infectious diseases, or inflammatory or autoimmune diseases; use of the anti-NKp46 antibody or antigen-binding fragment thereof according to any of the preceding claims in the treatment of cancers, infectious diseases, or inflammatory or autoimmune diseases; preferably, the cancers include: leukemia, aggressive lymphoma, non-Hodgkin lymphoma, glioma, cervical cancer, head and neck cancer, rectal cancer, kidney cancer, liver cancer, lung cancer, pancreatic cancer, stomach cancer, etc.

14. Use of the anti-NKp46 antibody or antigen-binding fragment thereof according to any of the preceding claims in the preparation of diagnostic reagents for NKp46-expressing.

15. A solution preparation, comprising the anti-NKp46 antibody or antigen-binding fragment thereof according to any of the preceding claims, and buffer solution.

16. A method for identifying the presence of NKp46-expressing cells in an individual, comprising obtaining a biological sample from the individual containing cells, contacting the cells with the anti-NKp46 antibody or its antigen-binding fragment thereof according to any of claims 1 to 5, and assessing the binding of the antibody to the cells or not.

17. A pharmaceutical composition comprising an effective amount of the anti- NKp46 antibody or antigen-binding fragment thereof according to any of the preceding claims 1 to 5, or an effective amount of the conjugate according to claim 6, or an effective amount of the bispecific antibody or the multi-specific antibody according to claim 7, or an effective amount of the antibody-drug conjugate of claim 8, or an effective amount of the chimeric antigen receptor according to claim 9, or an effective amount of the nucleic acid, the recombinant vector, or the host cell according to claim 10;
preferably, further comprising a pharmaceutically acceptable carrier;
preferably, further composing one or more additional therapeutic agents; preferably, the one or more additional therapeutic agents include: chemotherapeutic agents, cytotoxic agents, radiotherapeutic agents, cancer vaccines, anti-neoplastic agents, targeted anti-cancer agents, antiangiogenic agents, biological response modifiers, cytokines, hormones, anti-metastatic agents, and immunotherapeutic agents.
